# EUROPEAN PATENT SPECIFICATION

(11) **EP 1 774 005 B1**
(45) Date of publication and mention of the grant of the patent: **01.07.2009**
(21) Application number: 05773830.4
(22) Date of filing: 01.07.2005
(51) Int. Cl.: C12N 15/82

(54) **METHOD FOR ENHANCING GENE EXPRESSION IN PLANTS**
VERFAHREN ZUR VERBESSERUNG DER GENEXPRESSION IN PFLANZEN
PROCÉDÉ POUR AMÉLIORER L'EXPRESSION DE GÈNES DANS DES PLANTES

(30) Priority: 06.07.2004 EP 04103188
(43) Date of publication of application: 18.04.2007
(73) Proprietor: Biogemma, 75001 Paris (FR)
(72) Inventor: PAUL, Wyatt, Biogemma, F-63170 Aubiere (FR); RISACHER, Thierry, Biogemma UK, Cambridge CB4 0GZ (GB); LELONG, Baptiste, Biogemma UK, Cambridge CB4 0GZ (GB)
(74) Representative: Flesselles, Bruno F.G.
(86) International application number: PCT/EP2005/053148
(87) International publication number: WO 2006/003186

(56) References cited:
- WO-A-00/07430
- WO-A-91/09948
- WO-A-03/080802
- WO-A-20/04044205
- WO-A-20/04071168
- WO-A-20/04076616
- US-A- 5 593 874
- US-A1- 2004 126 845
- SUH, M C ET AL.: "Intron in 5 UTR of microsomal oleic acid desaturase (FAD2) gene is essential for enhancement of gene expression" PLANT BIOLOGY 2003, HONOLULU, HAWAII USA, THE AMERICAN SOCIETY OF PLANT BIOLOGISTS, [Online] 25 July 2003 (2003-07-25), XP002311900 Retrieved from the Internet: URL:http://abstracts.aspb.org/pb2003/publi c/P54/0732.html> [retrieved on 2004-12-22]
- OKULEY J ET AL: "ARABIDOPSIS FAD2 GENE ENCODES THE ENZYME THAT IS ESSENTIAL FOR POLYUNSATURATED LIPID SYNTHESIS" PLANT CELL, AMERICAN SOCIETY OF PLANT PHYSIOLOGISTS, ROCKVILLE, MD, US, vol. 6, 1994, pages 147-158, XP002034147 ISSN: 1040-4651
- PIRTLE IRMA L ET AL: "Molecular cloning and functional expression of the gene for a cotton DELTA-12 fatty acid desaturase (FAD2)" BIOCHIMICA ET BIOPHYSICA ACTA, vol. 1552, no. 2, 3 December 2001 (2001-12-03), pages 122-129, XP002311898 ISSN: 0006-3002
- MCELROY ET AL: "Construction of expression vectors based on the rice actin 1 (Act1) 5' region for use in monocot transformation" MOL. GEN. GENET, XX, XX, vol. 231, 1991, pages 150-160, XP002118811
- LIU QING ET AL: "Evolution of the FAD2-1 fatty acid desaturase 5' UTR intron and the molecular systematics of Gossypium (Malvaceae)" AMERICAN JOURNAL OF BOTANY, vol. 88, no. 1, January 2001 (2001-01), pages 92-102, XP002311897 ISSN: 0002-9122

### SEQUENCE LISTING

<110> BIOGEMMA
<120> Method for enhancing gene expression in plants
<130> BGM 29 - WO
<150> EP 04103188.1
   <151> 2004-07-06
<160> 12
<170> PatentIn version 3.2
<210> 1
   <211> 1135
   <212> DNA
   <213> Arabidopsis thaliana
<400> 1
   gtccgtcgct tctcttccat ttcttctcat tttcgatttt gattcttatt tctttccagt 60
   agctcctgct ctgtgaattt ctccgctcac gatagatctg cttatactcc ttacattcaa 120
   ccttagatct ggtctcgatt ctctgtttct ctgttttttt cttttggtcg agaatctgat 180
   gtttgtttat gttctgtcac cattaataat aatgaactct ctcattcata caatgattag 240
   tttctctcgt ctacaaaacg atatgttgca ttttcacttt tcttcttttt ttctaagatg 300
   atttgctttg accaatttgt ttagatcttt attctatttt attttctggt gggttggtgg 360
   aaattgaaaa aaaaaaaaac agcataaatt gttatttgtt aatgtattca ttttttggct 420
   atttgttctg ggtaaaaatc tgcttctact attgaatctt tcctggattt tttactccta 480
   ttgggttttt atagtaaaaa tacataataa aaggaaaaca aaagttttat agattctctt 540
   aaacccctta cgataaaagt tggaatcaaa ataattcagg atcagatgct ctttgattga 600
   ttcagatgcg attacagttg catggcaaat tttctagatc cgtcgtcaca ttttattttc 660
   tgtttaaata tctaaatctg atatatgatg tcgatcgaca aattctggtg gcttatacat 720
   cacttcaact gttttctttt ggctttgttt gtcaacttgg ttttcaatac gatttgtgat 780
   ttcgatcgct gaatttttaa tacaagcaaa ctgatgttaa ccacaagcaa gagatgtgac 840
   ctgccttatt aacatcgtat tacttactac tagtcgtatt ctcaacgcaa tcgtttttgt 900
   atttctcaca ttatgccgct tctctactct ttattccttt tggtccacgc attttctatt 960
   tgtggcaatc cctttcacaa cctgatttcc cactttggat catttgtctg aagactctct 1020
   tgaatcgtta ccacttgttt cttgtgcatg ctctgttttt tagaattaat gataaaacta 1080
   ttccatagtc ttgagttttc agcttgttga ttcttttgct tttggttttc tgcag 1135
<210> 2
   <211> 3391
   <212> DNA
   <213> Artificial
<220>
   <223> proVir35Sx2 - EcGUS - terVirCaMV
<220>
   <221> promoter
   <222> (1) .. (733)
   <223> 35 S x 2
<220>
   <221> Coding_sequenee
   <222> (782) .. (2587)
   <223> GUS protein
<220>
   <221> misc_feature
   <222> (2645)..(2646)
   <223> n is a, c, g, or t
<220>
   <221> terminator
   <222> (2657)..(3391)
<400> 2
   ctactccaaa aatgtcaaag atacagtctc agaagaccaa agggctattg agacttttca      60
   acaaagggta atttcgggaa acctcctcgg attccattgc ccagctatct gtcacttcat     120
   cgaaaggaca gtagaaaagg aaggtggctc ctacaaatgc catcattgcg ataaaggaaa     180
   ggctatcatt caagatgcct ctgccgacag tggtcccaaa gatggacccc cacccacgag     240
   gagcatcgtg gaaaaagaag acgttccaac cacgtcttca aagcaagtgg attgatgtga     300
   catctccact gacgtaaggg atgacgcaca atcccacccc tactccaaaa atgtcaaaga     360
   tacagtctca gaagaccaaa gggctattga gacttttcaa caaagggtaa tttcgggaaa     420
   cctcctcgga ttccattgcc cagctatctg tcacttcatc gaaaggacag tagaaaagga     480
   aggtggctcc tacaaatgcc atcattgcga taaaggaaag gctatcattc aagatgcctc     540
   tgccgacagt ggtcccaaag atggaccccc acccacgagg agcatcgtgg aaaaagaaga     600
   cgttccaacc acgtcttcaa agcaagtgga ttgatgtgac atctccactg acgtaaggga     660
   tgacgcacaa tcccactatc cttcgcaaga cccttcctct atataaggaa gttcatttca     720
   tttggagagg acagcccaag cttggctgca ggtcgacgga tccccgggtg gtcagtccct     780
   tatgttacgt cctgtagaaa ccccaacccg tgaaatcaaa aaactcgacg gcctgtgggc     840
   attcagtctg gatcgcgaaa actgtggaat tgatcagcgt tggtgggaaa gcgcgttaca     900
   agaaagccgg gcaattgctg tgccaggcag ttttaacgat cagttcgccg atgcagatat     960
   tcgtaattat gcgggcaacg tctggtatca gcgcgaagtc tttataccga aaggttgggc    1020
   aggccagcgt atcgtgctgc gtttcgatgc ggtcactcat tacggcaaag tgtgggtcaa 1080
   taatcaggaa gtgatggagc atcagggcgg ctatacgcca tttgaagccg atgtcacgcc 1140
   gtatgttatt gccgggaaaa gtgtacgtat caccgtttgt gtgaacaacg aactgaactg 1200
   gcagactatc ccgccgggaa tggtgattac cgacgaaaac ggcaagaaaa agcagtctta 1260
   cttccatgat ttctttaact atgccggaat ccatcgcagc gtaatgctct acaccacgcc 1320
   gaacacctgg gtggacgata tcaccgtggt gacgcatgtc gcgcaagact gtaaccacgc 1380
   gtctgttgac tggcaggtgg tggccaatgg tgatgtcagc gttgaactgc gtgatgcgga 1440
   tcaacaggtg gttgcaactg gacaaggcac tagcgggact ttgcaagtgg tgaatccgca 1500
   cctctggcaa ccgggtgaag gttatctcta tgaactgtgc gtcacagcca aaagccagac 1560
   agagtgtgat atctacccgc ttcgcgtcgg catccggtca gtggcagtga agggccaaca 1620
   gttcctgatt aaccacaaac cgttctactt tactggcttt ggtcgtcatg aagatgcgga 1680
   cttacgtggc aaaggattcg ataacgtgct gatggtgcac gaccacgcat taatggactg 1740
   gattggggcc aactcctacc gtacctcgca ttacccttac gctgaagaga tgctcgactg 1800
   ggcagatgaa catggcatcg tggtgattga tgaaactgct gctgtcggct ttaacctctc 1860
   tttaggcatt ggtttcgaag cgggcaacaa gccgaaagaa ctgtacagcg aagaggcagt 1920
   caacggggaa actcagcaag cgcacttaca ggcgattaaa gagctgatag cgcgtgacaa 1980
   aaaccaccca agcgtggtga tgtggagtat tgccaacgaa ccggataccc gtccgcaagt 2040
   gcacgggaat atttcgccac tggcggaagc aacgcgtaaa ctcgacccga cgcgtccgat 2100
   cacctgcgtc aatgtaatgt tctgcgacgc tcacaccgat accatcagcg atctctttga 2160
   tgtgctgtgc ctgaaccgtt attacggatg gtatgtccaa agcggcgatt tggaaacggc 2220
   agagaaggta ctggaaaaag aacttctggc ctggcaggag aaactgcatc agccgattat 2280
   catcaccgaa tacggcgtgg atacgttagc cgggctgcac tcaatgtaca ccgacatgtg 2340
   gagtgaagag tatcagtgtg catggctgga tatgtatcac cgcgtctttg atcgcgtcag 2400
   cgccgtcgtc ggtgaacagg tatggaattt cgccgatttt gcgacctcgc aaggcatatt 2460
   gcgcgttggc ggtaacaaga aagggatctt cactcgcgac cgcaaaccga agtcggcggc 2520
   ttttctgctg caaaaacgct ggactggcat gaacttcggt gaaaaaccgc agcagggagg 2580
   caaacaatga atcaacaact ctcctggcgc accatcgtcg gctacagcct cgggaattgc 2640
   taccnntcta gaattcggct gaaatcacca gtctctctct acaaatctat ctctctctat 2700
   tttctccata aataatgtgt gagtagtttc ccgataaggg aaattagggt tcttataggg 2760
   tttcgctcat gtgttgagca tataagaaac ccttagtatg tatttgtatt tgtaaaatac 2820
   ttctatcaat aaaatttcta attcctaaaa ccaaaatcca gtactaaaat ccagatctcc   2880
   taaagtccct atagatcttt gtcgtgaata taaaccagac acgagacgac taaacctgga   2940
   gcccagacgc cgttcgaagc tagaagtacc gcttaggcag gaggccgtta gggaaaagat   3000
   gctaaggcag ggttggttac gttgactccc ccgtaggttt ggtttaaata tgatgaagtg   3060
   gacggaagga aggaggaaga caaggaagga taaggttgca ggccctgtgc aaggtaagaa   3120
   gatggaaatt tgatagaggt acgctactat acttatacta tacgctaagg gaatgcttgt   3180
   atttataccc tataccccct aataacccct tatcaattta agaaataatc cgcataagcc   3240
   cccgcttaaa aattggtatc agagccatga ataggtctat gaccaaaact caagaggata   3300
   aaacctcacc aaaatacgaa agagttctta actctaaaga taaaagatct ttcaagatca   3360
   aaactagttc cctcacaccg gtgacgggga t                                  3391
<210> 3
   <211> 4534
   <212> DNA
   <213> artificial
<220>
   <223> proVir35Sx2 - intAtFAD2 - EcGUS - terVirCaMV
<220>
   <221> promoter
   <222> (1)..(733)
   <223> 35 S x 2
<220>
   <221> Intron
   <222> (772)..(1902)
   <223> FAD 2 intron
<220>
   <221> Coding_sequence
   <222> (1925)..(3730)
   <223> GUS protein
<220>
   <221> misc_feature
   <222> (3788)..(3789)
   <223> n is a, c, g, or t
<220>
   <221> terminator
   <222> (3800)..(4534)
   <223> CAMV terminator
<400> 3
   ctactccaaa aatgtcaaag atacagtctc agaagaccaa agggctattg agacttttca    60
   acaaagggta atttcgggaa acctcctcgg attccattgc ccagctatct gtcacttcat   120
   cgaaaggaca gtagaaaagg aaggtggctc ctacaaatgc catcattgcg ataaaggaaa   180
   ggctatcatt caagatgcct ctgccgacag tggtcccaaa gatggacccc cacccacgag 240
   gagcatcgtg gaaaaagaag acgttccaac cacgtcttca aagcaagtgg attgatgtga 300
   catctccact gacgtaaggg atgacgcaca atcccacccc tactccaaaa atgtcaaaga 360
   tacagtctca gaagaccaaa gggctattga gacttttcaa caaagggtaa tttcgggaaa 420
   cctcctcgga ttccattgcc cagctatctg tcacttcatc gaaaggacag tagaaaagga 480
   aggtggctcc tacaaatgcc atcattgcga taaaggaaag gctatcattc aagatgcctc 540
   tgccgacagt ggtcccaaag atggaccccc acccacgagg agcatcgtgg aaaaagaaga 600
   cgttccaacc acgtcttcaa agcaagtgga ttgatgtgac atctccactg acgtaaggga 660
   tgacgcacaa tcccactatc cttcgcaaga cccttcctct atataaggaa gttcatttca 720
   tttggagagg acagcccaag cttggctgca ggtcgacgga tccccgggtt tgtccgtcgc 780
   ttctcttcca tttcttctca ttttcgattt tgattcttat ttctttccag tagctcctgc 840
   tctgtgaatt tctccgctca cgatagatct gcttatactc cttacattca accttagatc 900
   tggtctcgat tctctgtttc tctgtttttt tcttttggtc gagaatctga tgtttgttta 960
   tgttctgtca ccattaataa taatgaactc tctcattcat acaatgatta gtttctctcg 1020
   tctacaaaac gatatgttgc attttcactt ttcttctttt tttctaagat gatttgcttt 1080
   gaccaatttg tttagatctt tattctattt tattttctgg tgggttggtg gaaattgaaa 1140
   aaaaaaaaaa cagcataaat tgttatttgt taatgtattc attttttggc tatttgttct 1200
   gggtaaaaat ctgcttctac tattgaatct ttcctggatt ttttactcct attgggtttt 1260
   tatagtaaaa atacataata aaaggaaaac aaaagtttta tagattctct taaacccctt 1320
   acgataaaag ttggaatcaa aataattcag gatcagatgc tctttgattg attcagatgc 1380
   gattacagtt gcatggcaaa ttttctagat ccgtcgtcac attttatttt ctgtttaaat 1440
   atctaaatct gatatatgat gtcgacaaat tctggtggct tatacatcac ttcaactgtt 1500
   ttcttttggc tttgtttgtc aacttggttt tcaatacgat ttgtgatttc gatcgctgaa 1560
   tttttaatac aagcaaactg atgttaacca caagcaagag atgtgacctg ccttattaac 1620
   atcgtattac ttactactag tcgtattctc aacgcaatcg tttttgtatt tctcacatta 1680
   tgccgcttct ctactcttta ttccttttgg tccacgcatt ttctatttgt ggcaatccct 1740
   ttcacaacct gatttcccac tttggatcat ttgtctgaag actctcttga atcgttacca 1800
   cttgtttctt gtgcatgctc tgttttttag aattaatgat aaaactattc catagtcttg 1860
   agttttcagc ttgttgattc ttttgctttt ggttttctgc agtggcccgg gtggtcagtc 1920
   ccttatgtta cgtcctgtag aaaccccaac ccgtgaaatc aaaaaactcg acggcctgtg 1980
   ggcattcagt ctggatcgcg aaaactgtgg aattgatcag cgttggtggg aaagcgcgtt 2040
   acaagaaagc cgggcaattg ctgtgccagg cagttttaac gatcagttcg ccgatgcaga 2100
   tattcgtaat tatgcgggca acgtctggta tcagcgcgaa gtctttatac cgaaaggttg 2160
   ggcaggccag cgtatcgtgc tgcgtttcga tgcggtcact cattacggca aagtgtgggt 2220
   caataatcag gaagtgatgg agcatcaggg cggctatacg ccatttgaag ccgatgtcac 2280
   gccgtatgtt attgccggga aaagtgtacg tatcaccgtt tgtgtgaaca acgaactgaa 2340
   ctggcagact atcccgccgg gaatggtgat taccgacgaa aacggcaaga aaaagcagtc 2400
   ttacttccat gatttcttta actatgccgg aatccatcgc agcgtaatgc tctacaccac 2460
   gccgaacacc tgggtggacg atatcaccgt ggtgacgcat gtcgcgcaag actgtaacca 2520
   cgcgtctgtt gactggcagg tggtggccaa tggtgatgtc agcgttgaac tgcgtgatgc 2580
   ggatcaacag gtggttgcaa ctggacaagg cactagcggg actttgcaag tggtgaatcc 2640
   gcacctctgg caaccgggtg aaggttatct ctatgaactg tgcgtcacag ccaaaagcca 2700
   gacagagtgt gatatctacc cgcttcgcgt cggcatccgg tcagtggcag tgaagggcca 2760
   acagttcctg attaaccaca aaccgttcta ctttactggc tttggtcgtc atgaagatgc 2820
   ggacttacgt ggcaaaggat tcgataacgt gctgatggtg cacgaccacg cattaatgga 2880
   ctggattggg gccaactcct accgtacctc gcattaccct tacgctgaag agatgctcga 2940
   ctgggcagat gaacatggca tcgtggtgat tgatgaaact gctgctgtcg gctttaacct 3000
   ctctttaggc attggtttcg aagcgggcaa caagccgaaa gaactgtaca gcgaagaggc 3060
   agtcaacggg gaaactcagc aagcgcactt acaggcgatt aaagagctga tagcgcgtga 3120
   caaaaaccac ccaagcgtgg tgatgtggag tattgccaac gaaccggata cccgtccgca 3180
   agtgcacggg aatatttcgc cactggcgga agcaacgcgt aaactcgacc cgacgcgtcc 3240
   gatcacctgc gtcaatgtaa tgttctgcga cgctcacacc gataccatca gcgatctctt 3300
   tgatgtgctg tgcctgaacc gttattacgg atggtatgtc caaagcggcg atttggaaac 3360
   ggcagagaag gtactggaaa aagaacttct ggcctggcag gagaaactgc atcagccgat 3420
   tatcatcacc gaatacggcg tggatacgtt agccgggctg cactcaatgt acaccgacat 3480
   gtggagtgaa gagtatcagt gtgcatggct ggatatgtat caccgcgtct ttgatcgcgt 3540
   cagcgccgtc gtcggtgaac aggtatggaa tttcgccgat tttgcgacct cgcaaggcat 3600
   attgcgcgtt ggcggtaaca agaaagggat cttcactcgc gaccgcaaac cgaagtcggc 3660
   ggcttttctg ctgcaaaaac gctggactgg catgaacttc ggtgaaaaac cgcagcaggg 3720
   aggcaaacaa tgaatcaaca actctcctgg cgcaccatcg tcggctacag cctcgggaat 3780
   tgctaccnnt ctagaattcg gctgaaatca ccagtctctc tctacaaatc tatctctctc 3840
   tattttctcc ataaataatg tgtgagtagt ttcccgataa gggaaattag ggttcttata   3900
   gggtttcgct catgtgttga gcatataaga aacccttagt atgtatttgt atttgtaaaa   3960
   tacttctatc aataaaattt ctaattccta aaaccaaaat ccagtactaa aatccagatc   4020
   tcctaaagtc cctatagatc tttgtcgtga atataaacca gacacgagac gactaaacct   4080
   ggagcccaga cgccgttcga agctagaagt accgcttagg caggaggccg ttagggaaaa   4140
   gatgctaagg cagggttggt tacgttgact cccccgtagg tttggtttaa atatgatgaa   4200
   gtggacggaa ggaaggagga agacaaggaa ggataaggtt gcaggccctg tgcaaggtaa   4260
   gaagatggaa atttgataga ggtacgctac tatacttata ctatacgcta agggaatgct   4320
   tgtatttata ccctataccc cctaataacc ccttatcaat ttaagaaata atccgcataa   4380
   gcccccgctt aaaaattggt atcagagcca tgaataggtc tatgaccaaa actcaagagg   4440
   ataaaacctc accaaaatac gaaagagttc ttaactctaa agataaaaga tctttcaaga   4500
   tcaaaactag ttccctcaca ccggtgacgg ggat                                4534
<210> 4
   <211> 3361
   <212> DNA
   <213> artificial
<220>
   <223> proVirSc4 - EcGUSintStLS1 - terVirCaMV
<220>
   <221> promoter
   <222> (1)..(532)
   <223> Sc4 promoter
<220>
   <221> Coding_sequence
   <222> (551)..(2545)
   <223> Gus protein
<220>
   <221> terminator
   <222> (2578)..(3361)
   <223> CAMV terminator
<400> 4
   taattgttat tatcaataaa agaattttta ttgttattgt gttatttggt aatttatgct      60
   tataagtaat tctatgatta attgtgaatt aataagacta atgaggataa taattgaatt     120
   tgattaaatt aactctgcga agccatatgt ctttcacgtg agagtcacgt gatgtctccg     180
   cgacaggctg gcacggggct tagtattacc cccgtgccgg gatcagagac atttgactaa     240
   atgttgactt ggaataatag cccttggatt agatgacacg tggacgctca ggatctgtga     300
   tgctagtgaa gcgcttaagc tgaacgaatc tgacggaaga gcggacaaac gcacatggac 360
   tatggcccac tgctttatta aagaagtgaa tgacagctgt ctttgcttca agacgaagta 420
   aagaatagtg gaaaacgcgt aaagaataag cgtactcagt acgcttcgtg gctttataaa 480
   tagtgcttcg tcttattctt cgttgtatca tcaacgaaga agttaagctg atcccgggaa 540
   ttcgcggccc atggtacgtc ctgtagaaac cccaacccgt gaaatcaaaa aactcgacgg 600
   cctgtgggca ttcagtctgg atcgcgaaaa ctgtggaatt gatcagcgtt ggtgggaaag 660
   cgcgttacaa gaaagccggg caattgctgt gccaggcagt tttaacgatc agttcgccga 720
   tgcagatatt cgtaattatg cgggcaacgt ctggtatcag cgcgaagtct ttataccgaa 780
   aggttgggca ggccagcgta tcgtgctgcg tttcgatgcg gtcactcatt acggcaaagt 840
   gtgggtcaat aatcaggaag tgatggagca tcagggcggc tatacgccat ttgaagccga 900
   tgtcacgccg tatgttattg ccgggaaaag tgtacgtaag tttctgcttc tacctttgat 960
   atatatataa taattatcat taattagtag taatataata tttcaaatat ttttttcaaa 1020
   ataaaagaat gtagtatata gcaattgctt ttctgtagtt tataagtgtg tatattttaa 1080
   tttataactt ttctaatata tgaccaaaat ttgttgatgt gcaggtatca ccgtttgtgt 1140
   gaacaacgaa ctgaactggc agactatccc gccgggaatg gtgattaccg acgaaaacgg 1200
   caagaaaaag cagtcttact tccatgattt ctttaactat gccggaatcc atcgcagcgt 1260
   aatgctctac accacgccga acacctgggt ggacgatatc accgtggtga cgcatgtcgc 1320
   gcaagactgt aaccacgcgt ctgttgactg gcaggtggtg gccaatggtg atgtcagcgt 1380
   tgaactgcgt gatgcggatc aacaggtggt tgcaactgga caaggcacta gcgggacttt 1440
   gcaagtggtg aatccgcacc tctggcaacc gggtgaaggt tatctctatg aactgtgcgt 1500
   cacagccaaa agccagacag agtgtgatat ctacccgctt cgcgtcggca tccggtcagt 1560
   ggcagtgaag ggccaacagt tcctgattaa ccacaaaccg ttctacttta ctggctttgg 1620
   tcgtcatgaa gatgcggact tacgtggcaa aggattcgat aacgtgctga tggtgcacga 1680
   ccacgcatta atggactgga ttggggccaa ctcctaccgt acctcgcatt acccttacgc 1740
   tgaagagatg ctcgactggg cagatgaaca tggcatcgtg gtgattgatg aaactgctgc 1800
   tgtcggcttt aacctctctt taggcattgg tttcgaagcg ggcaacaagc cgaaagaact 1860
   gtacagcgaa gaggcagtca acggggaaac tcagcaagcg cacttacagg cgattaaaga 1920
   gctgatagcg cgtgacaaaa accacccaag cgtggtgatg tggagtattg ccaacgaacc 1980
   ggatacccgt ccgcaagtgc acgggaatat ttcgccactg gcggaagcaa cgcgtaaact 2040
   cgacccgacg cgtccgatca cctgcgtcaa tgtaatgttc tgcgacgctc acaccgatac 2100
   catcagcgat ctctttgatg tgctgtgcct gaaccgttat tacggatggt atgtccaaag 2160
   cggcgatttg gaaacggcag agaaggtact ggaaaaagaa cttctggcct ggcaggagaa 2220
   actgcatcag ccgattatca tcaccgaata cggcgtggat acgttagccg ggctgcactc 2280
   aatgtacacc gacatgtgga gtgaagagta tcagtgtgca tggctggata tgtatcaccg 2340
   cgtctttgat cgcgtcagcg ccgtcgtcgg tgaacaggta tggaatttcg ccgattttgc 2400
   gacctcgcaa ggcatattgc gcgttggcgg taacaagaaa gggatcttca ctcgcgaccg 2460
   caaaccgaag tcggcggctt ttctgctgca aaaacgctgg actggcatga acttcggtga 2520
   aaaaccgcag cagggaggca aacaatgaat caacaactct cctggcgcac catcgtcggc 2580
   tacagcctcg gtgacgtcgg atcccccggg ctgcaggaat tcggtacgct gaaatcacca 2640
   gtctctctct acaaatctat ctctctctat tttctccata aataatgtgt gagtagtttc 2700
   ccgataaggg aaattagggt tcttataggg tttcgctcat gtgttgagca tataagaaac 2760
   ccttagtatg tatttgtatt tgtaaaatac ttctatcaat aaaatttcta attcctaaaa 2820
   ccaaaatcca gtactaaaat ccagatctcc taaagtccct atagatcttt gtcgtgaata 2880
   taaaccagac acgagacgac taaacctgga gcccagacgc cgttcgaagc tagaagtacc 2940
   gcttaggcag gaggccgtta gggaaaagat gctaaggcag ggttggttac gttgactccc 3000
   ccgtaggttt ggtttaaata tgatgaagtg gacggaagga aggaggaaga caaggaagga 3060
   taaggttgca ggccctgtgc aaggtaagaa gatggaaatt tgatagaggt acgctactat 3120
   acttatacta tacgctaagg gaatgcttgt atttataccc tataccccct aataacccct 3180
   tatcaattta agaaataatc cgcataagcc cccgcttaaa aattggtatc agagccatga 3240
   ataggtctat gaccaaaact caagaggata aaacctcacc aaaatacgaa agagttctta 3300
   actctaaaga taaaagatct ttcaagatca aaactagttc cctcacaccg gtgacgggga 3360
   t 3361
<210> 5
   <211> 4561
   <212> DNA
   <213> artificial
<220>
   <223> proVirSc4 - intAtFAD2 - EcGUSintStLS1 - terVirCaMV
<220>
   <221> promoter
   <222> (1) .. (532)
   <223> Sc4 promoter
<220>
   <221> Intron
   <222> (580)..(1714)
   <223> FAD 2 intron
<220>
   <221> coding_sequence
   <222> (1751)..(3745)
   <223> GUS protein
<220>
   <221> terminator
   <222> (3778) .. (4561)
   <223> CAMV terminator
<400> 5
   taattgttat tatcaataaa agaattttta ttgttattgt gttatttggt aatttatgct     60
   tataagtaat tctatgatta attgtgaatt aataagacta atgaggataa taattgaatt    120
   tgattaaatt aactctgcga agccatatgt ctttcacgtg agagtcacgt gatgtctccg    180
   cgacaggctg gcacggggct tagtattacc cccgtgccgg gatcagagac atttgactaa    240
   atgttgactt ggaataatag cccttggatt agatgacacg tggacgctca ggatctgtga    300
   tgctagtgaa gcgcttaagc tgaacgaatc tgacggaaga gcggacaaac gcacatggac    360
   tatggcccac tgctttatta aagaagtgaa tgacagctgt ctttgcttca agacgaagta    420
   aagaatagtg gaaaacgcgt aaagaataag cgtactcagt acgcttcgtg gctttataaa    480
   tagtgcttcg tcttattctt cgttgtatca tcaacgaaga agttaagctg atcccgggaa    540
   ttcgcggccg ctctagaact agtggatccc ccgggccagg tccgtcgctt ctcttccatt    600
   tcttctcatt ttcgattttg attcttattt ctttccagta gctcctgctc tgtgaatttc    660
   tccgctcacg atagatctgc ttatactcct tacattcaac cttagatctg gtctcgattc    720
   tctgtttctc tgtttttttc ttttggtcga gaatctgatg tttgtttatg ttctgtcacc    780
   attaataata atgaactctc tcattcatac aatgattagt ttctctcgtc tacaaaacga    840
   tatgttgcat tttcactttt cttctttttt tctaagatga tttgctttga ccaatttgtt    900
   tagatcttta ttctatttta ttttctggtg ggttggtgga aattgaaaaa aaaaaaaaca    960
   gcataaattg ttatttgtta atgtattcat tttttggcta tttgttctgg gtaaaaatct   1020
   gcttctacta ttgaatcttt cctggatttt ttactcctat tgggttttta tagtaaaaat   1080
   acataataaa aggaaaacaa aagttttata gattctctta aaccccttac gataaaagtt   1140
   ggaatcaaaa taattcagga tcagatgctc tttgattgat tcagatgcga ttacagttgc   1200
   atggcaaatt ttctagatcc gtcgtcacat tttattttct gtttaaatat ctaaatctga   1260
   tatatgatgt cgatcgacaa attctggtgg cttatacatc acttcaactg ttttcttttg   1320
   gctttgtttg tcaacttggt tttcaatacg atttgtgatt tcgatcgctg aatttttaat   1380
   acaagcaaac tgatgttaac cacaagcaag agatgtgacc tgccttatta acatcgtatt   1440
   acttactact agtcgtattc tcaacgcaat cgtttttgta tttctcacat tatgccgctt 1500
   ctctactctt tattcctttt ggtccacgca ttttctattt gtggcaatcc ctttcacaac 1560
   ctgatttccc actttggatc atttgtctga agactctctt gaatcgttac cacttgtttc 1620
   ttgtgcatgc tctgtttttt agaattaatg ataaaactat tccatagtct tgagttttca 1680
   gcttgttgat tcttttgctt ttggttttct gcagaaaccc gggctgcagg aattcgatat 1740
   caagcttccc atggtacgtc ctgtagaaac cccaacccgt gaaatcaaaa aactcgacgg 1800
   cctgtgggca ttcagtctgg atcgcgaaaa ctgtggaatt gatcagcgtt ggtgggaaag 1860
   cgcgttacaa gaaagccggg caattgctgt gccaggcagt tttaacgatc agttcgccga 1920
   tgcagatatt cgtaattatg cgggcaacgt ctggtatcag cgcgaagtct ttataccgaa 1980
   aggttgggca ggccagcgta tcgtgctgcg tttcgatgcg gtcactcatt acggcaaagt 2040
   gtgggtcaat aatcaggaag tgatggagca tcagggcggc tatacgccat ttgaagccga 2100
   tgtcacgccg tatgttattg ccgggaaaag tgtacgtaag tttctgcttc tacctttgat 2160
   atatatataa taattatcat taattagtag taatataata tttcaaatat ttttttcaaa 2220
   ataaaagaat gtagtatata gcaattgctt ttctgtagtt tataagtgtg tatattttaa 2280
   tttataactt ttctaatata tgaccaaaat ttgttgatgt gcaggtatca ccgtttgtgt 2340
   gaacaacgaa ctgaactggc agactatccc gccgggaatg gtgattaccg acgaaaacgg 2400
   caagaaaaag cagtcttact tccatgattt ctttaactat gccggaatcc atcgcagcgt 2460
   aatgctctac accacgccga acacctgggt ggacgatatc accgtggtga cgcatgtcgc 2520
   gcaagactgt aaccacgcgt ctgttgactg gcaggtggtg gccaatggtg atgtcagcgt 2580
   tgaactgcgt gatgcggatc aacaggtggt tgcaactgga caaggcacta gcgggacttt 2640
   gcaagtggtg aatccgcacc tctggcaacc gggtgaaggt tatctctatg aactgtgcgt 2700
   cacagccaaa agccagacag agtgtgatat ctacccgctt cgcgtcggca tccggtcagt 2760
   ggcagtgaag ggccaacagt tcctgattaa ccacaaaccg ttctacttta ctggctttgg 2820
   tcgtcatgaa gatgcggact tacgtggcaa aggattcgat aacgtgctga tggtgcacga 2880
   ccacgcatta atggactgga ttggggccaa ctcctaccgt acctcgcatt acccttacgc 2940
   tgaagagatg ctcgactggg cagatgaaca tggcatcgtg gtgattgatg aaactgctgc 3000
   tgtcggcttt aacctctctt taggcattgg tttcgaagcg ggcaacaagc cgaaagaact 3060
   gtacagcgaa gaggcagtca acggggaaac tcagcaagcg cacttacagg cgattaaaga 3120
   gctgatagcg cgtgacaaaa accacccaag cgtggtgatg tggagtattg ccaacgaacc 3180
   ggatacccgt ccgcaagtgc acgggaatat ttcgccactg gcggaagcaa cgcgtaaact 3240
   cgacccgacg cgtccgatca cctgcgtcaa tgtaatgttc tgcgacgctc acaccgatac 3300
   catcagcgat ctctttgatg tgctgtgcct gaaccgttat tacggatggt atgtccaaag 3360
   cggcgatttg gaaacggcag agaaggtact ggaaaaagaa cttctggcct ggcaggagaa 3420
   actgcatcag ccgattatca tcaccgaata cggcgtggat acgttagccg ggctgcactc 3480
   aatgtacacc gacatgtgga gtgaagagta tcagtgtgca tggctggata tgtatcaccg 3540
   cgtctttgat cgcgtcagcg ccgtcgtcgg tgaacaggta tggaatttcg ccgattttgc 3600
   gacctcgcaa ggcatattgc gcgttggcgg taacaagaaa gggatcttca ctcgcgaccg 3660
   caaaccgaag tcggcggctt ttctgctgca aaaacgctgg actggcatga acttcggtga 3720
   aaaaccgcag cagggaggca aacaatgaat caacaactct cctggcgcac catcgtcggc 3780
   tacagcctcg gtgacgtcgg atcccccggg ctgcaggaat tcggtacgct gaaatcacca 3840
   gtctctctct acaaatctat ctctctctat tttctccata aataatgtgt gagtagtttc 3900
   ccgataaggg aaattagggt tcttataggg tttcgctcat gtgttgagca tataagaaac 3960
   ccttagtatg tatttgtatt tgtaaaatac ttctatcaat aaaatttcta attcctaaaa 4020
   ccaaaatcca gtactaaaat ccagatctcc taaagtccct atagatcttt gtcgtgaata 4080
   taaaccagac acgagacgac taaacctgga gcccagacgc cgttcgaagc tagaagtacc 4140
   gcttaggcag gaggccgtta gggaaaagat gctaaggcag ggttggttac gttgactccc 4200
   ccgtaggttt ggtttaaata tgatgaagtg gacggaagga aggaggaaga caaggaagga 4260
   taaggttgca ggccctgtgc aaggtaagaa gatggaaatt tgatagaggt acgctactat 4320
   acttatacta tacgctaagg gaatgcttgt atttataccc tataccccct aataacccct 4380
   tatcaattta agaaataatc cgcataagcc cccgcttaaa aattggtatc agagccatga 4440
   ataggtctat gaccaaaact caagaggata aaacctcacc aaaatacgaa agagttctta 4500
   actctaaaga taaaagatct ttcaagatca aaactagttc cctcacaccg gtgacgggga 4560
   t 4561
<210> 6
   <211> 3307
   <212> DNA
   <213> artificial
<220>
   <223> proVirBSV - EcGUSintStLS1 - terVirCaMV
<220>
   <221> promoter
   <222> (1) .. (494)
   <223> BSV promoter
<220>
   <221> Coding_sequence
   <222> (497) .. (2491)
   <223> GUS protein
<220>
   <221> terminator
   <222> (2571) .. (3307)
   <223> CAMV terminator
<400> 6
   gatgctctag atctgctgga tatcagtaat gatgactgaa gcggaagtgg cggaccccta     60
   ccacgtgttg ataccaaccg gtgtgaagac tgataagatg cggagtgagc tggataccac    120
   tcactttatg taaagaggag acaaagtata atgtctcttt attttaagtt tgtcggtgtg    180
   cgttgtctag tcacgcacga tgacctttag tgactttgca ggattcttac gcaaagttgt    240
   taggccagag acatgtgatg atgcttatct gcattattgg tggatgccac ctaacgatgc    300
   cagaaagctc cacaactctc tatataagga gccttgtatt caggttgcaa acacgcacca    360
   caacgcgagt ttactcctga tttgagaaat aaaaacttct gtgcttgaaa cacactttgt    420
   gcgagttcac tttgtgcgag tagagcgcaa gatcctagtt ccgcgagcgt agacccgtct    480
   ggtatcagag caagccatgg tacgtcctgt agaaacccca acccgtgaaa tcaaaaaact    540
   cgacggcctg tgggcattca gtctggatcg cgaaaactgt ggaattgatc agcgttggtg    600
   ggaaagcgcg ttacaagaaa gccgggcaat tgctgtgcca ggcagtttta acgatcagtt    660
   cgccgatgca gatattcgta attatgcggg caacgtctgg tatcagcgcg aagtctttat    720
   accgaaaggt tgggcaggcc agcgtatcgt gctgcgtttc gatgcggtca ctcattacgg    780
   caaagtgtgg gtcaataatc aggaagtgat ggagcatcag ggcggctata cgccatttga    840
   agccgatgtc acgccgtatg ttattgccgg gaaaagtgta cgtaagtttc tgcttctacc    900
   tttgatatat atataataat tatcattaat tagtagtaat ataatatttc aaatattttt    960
   ttcaaaataa aagaatgtag tatatagcaa ttgcttttct gtagtttata agtgtgtata   1020
   ttttaattta taacttttct aatatatgac caaaatttgt tgatgtgcag gtatcaccgt   1080
   ttgtgtgaac aacgaactga actggcagac tatcccgccg ggaatggtga ttaccgacga   1140
   aaacggcaag aaaaagcagt cttacttcca tgatttcttt aactatgccg gaatccatcg   1200
   cagcgtaatg ctctacacca cgccgaacac ctgggtggac gatatcaccg tggtgacgca   1260
   tgtcgcgcaa gactgtaacc acgcgtctgt tgactggcag gtggtggcca atggtgatgt   1320
   cagcgttgaa ctgcgtgatg cggatcaaca ggtggttgca actggacaag gcactagcgg   1380
   gactttgcaa gtggtgaatc cgcacctctg gcaaccgggt gaaggttatc tctatgaact   1440
   gtgcgtcaca gccaaaagcc agacagagtg tgatatctac ccgcttcgcg tcggcatccg   1500
   gtcagtggca gtgaagggcc aacagttcct gattaaccac aaaccgttct actttactgg   1560
   ctttggtcgt catgaagatg cggacttacg tggcaaagga ttcgataacg tgctgatggt 1620
   gcacgaccac gcattaatgg actggattgg ggccaactcc taccgtacct cgcattaccc 1680
   ttacgctgaa gagatgctcg actgggcaga tgaacatggc atcgtggtga ttgatgaaac 1740
   tgctgctgtc ggctttaacc tctctttagg cattggtttc gaagcgggca acaagccgaa 1800
   agaactgtac agcgaagagg cagtcaacgg ggaaactcag caagcgcact tacaggcgat 1860
   taaagagctg atagcgcgtg acaaaaacca cccaagcgtg gtgatgtgga gtattgccaa 1920
   cgaaccggat acccgtccgc aagtgcacgg gaatatttcg ccactggcgg aagcaacgcg 1980
   taaactcgac ccgacgcgtc cgatcacctg cgtcaatgta atgttctgcg acgctcacac 2040
   cgataccatc agcgatctct ttgatgtgct gtgcctgaac cgttattacg gatggtatgt 2100
   ccaaagcggc gatttggaaa cggcagagaa ggtactggaa aaagaacttc tggcctggca 2160
   ggagaaactg catcagccga ttatcatcac cgaatacggc gtggatacgt tagccgggct 2220
   gcactcaatg tacaccgaca tgtggagtga agagtatcag tgtgcatggc tggatatgta 2280
   tcaccgcgtc tttgatcgcg tcagcgccgt cgtcggtgaa caggtatgga atttcgccga 2340
   ttttgcgacc tcgcaaggca tattgcgcgt tggcggtaac aagaaaggga tcttcactcg 2400
   cgaccgcaaa ccgaagtcgg cggcttttct gctgcaaaaa cgctggactg gcatgaactt 2460
   cggtgaaaaa ccgcagcagg gaggcaaaca atgaatcaac aactctcctg gcgcaccatc 2520
   gtcggctaca gcctcggtga cgtcggatcc cccgggctgc aggaattcgg tacgctgaaa 2580
   tcaccagtct ctctctacaa atctatctct ctctattttc tccataaata atgtgtgagt 2640
   agtttcccga taagggaaat tagggttctt atagggtttc gctcatgtgt tgagcatata 2700
   agaaaccctt agtatgtatt tgtatttgta aaatacttct atcaataaaa tttctaattc 2760
   ctaaaaccaa aatccagtac taaaatccag atctcctaaa gtccctatag atctttgtcg 2820
   tgaatataaa ccagacacga gacgactaaa cctggagccc agacgccgtt cgaagctaga 2880
   agtaccgctt aggcaggagg ccgttaggga aaagatgcta aggcagggtt ggttacgttg 2940
   actcccccgt aggtttggtt taaatatgat gaagtggacg gaaggaagga ggaagacaag 3000
   gaaggataag gttgcaggcc ctgtgcaagg taagaagatg gaaatttgat agaggtacgc 3060
   tactatactt atactatacg ctaagggaat gcttgtattt ataccctata ccccctaata 3120
   accccttatc aatttaagaa ataatccgca taagcccccg cttaaaaatt ggtatcagag 3180
   ccatgaatag gtctatgacc aaaactcaag aggataaaac ctcaccaaaa tacgaaagag 3240
   ttcttaactc taaagataaa agatctttca agatcaaaac tagttccctc acaccggtga 3300
   cggggat 3307
<210> 7
   <211> 4259
   <212> DNA
   <213> artificial
<220>
   <223> provirBSV - intAtFAD2 - EcGUS - terVirCaMV
<220>
   <221> promoter
   <222> (1)..(475)
   <223> BSV promoter
<220>
   <221> Intron
   <222> (497)..(1627)
   <223> FAD 2 intron
<220>
   <221> Coding_sequence
   <222> (1650)..(3455)
   <223> GUS protein
<220>
   <221> misc_feature
   <222> (3513) .. (3514)
   <223> n is a, c, g, or t
<220>
   <221> terminator
   <222> (3525)..(4259)
   <223> GUS protein
<400> 7
   tctagatctg ctggatatca gtaatgatga ctgaagcgga agtggcggac ccctaccacg 60
   tgttgatacc aaccggtgtg aagactgata agatgcggag tgagctggat accactcact 120
   ttatgtaaag aggagacaaa gtataatgtc tctttatttt aagtttgtcg gtgtgcgttg 180
   tctagtcacg cacgatgacc tttagtgact ttgcaggatt cttacgcaaa gttgttaggc 240
   cagagacatg tgatgatgct tatctgcatt attggtggat gccacctaac gatgccagaa 300
   agctccacaa ctctctatat aaggagcctt gtattcaggt tgcaaacacg caccacaacg 360
   cgagtttact cctgatttga gaaataaaaa cttctgtgct tgaaacacac tttgtgcgag 420
   ttcactttgt gcgagtagag cgcaagatcc tagttccgcg agcgtagacc cgtctgtcga 480
   cggatccccg ggccaggtcc gtcgcttctc ttccatttct tctcattttc gattttgatt 540
   cttatttctt tccagtagct cctgctctgt gaatttctcc gctcacgata gatctgctta 600
   tactccttac attcaacctt agatctggtc tcgattctct gtttctctgt ttttttcttt 660
   tggtcgagaa tctgatgttt gtttatgttc tgtcaccatt aataataatg aactctctca 720
   ttcatacaat gattagtttc tctcgtctac aaaacgatat gttgcatttt cacttttctt 780
   ctttttttct aagatgattt gctttgacca atttgtttag atctttattc tattttattt 840
   tctggtgggt tggtggaaat tgaaaaaaaa aaaaacagca taaattgtta tttgttaatg 900
   tattcatttt ttggctattt gttctgggta aaaatctgct tctactattg aatctttcct 960
   ggatttttta ctcctattgg gtttttatag taaaaataca taataaaagg aaaacaaaag 1020
   ttttatagat tctcttaaac cccttacgat aaaagttgga atcaaaataa ttcaggatca 1080
   gatgctcttt gattgattca gatgcgatta cagttgcatg gcaaattttc tagatccgtc 1140
   gtcacatttt attttctgtt taaatatcta aatctgatat atgatgtcga caaattctgg 1200
   tggcttatac atcacttcaa ctgttttctt ttggctttgt ttgtcaactt ggttttcaat 1260
   acgatttgtg atttcgatcg ctgaattttt aatacaagca aactgatgtt aaccacaagc 1320
   aagagatgtg acctgcctta ttaacatcgt attacttact actagtcgta ttctcaacgc 1380
   aatcgttttt gtatttctca cattatgccg cttctctact ctttattcct tttggtccac 1440
   gcattttcta tttgtggcaa tccctttcac aacctgattt cccactttgg atcatttgtc 1500
   tgaagactct cttgaatcgt taccacttgt ttcttgtgca tgctctgttt tttagaatta 1560
   atgataaaac tattccatag tcttgagttt tcagcttgtt gattcttttg cttttggttt 1620
   tctgcagaaa cccgggtggt cagtccctta tgttacgtcc tgtagaaacc ccaacccgtg 1680
   aaatcaaaaa actcgacggc ctgtgggcat tcagtctgga tcgcgaaaac tgtggaattg 1740
   atcagcgttg gtgggaaagc gcgttacaag aaagccgggc aattgctgtg ccaggcagtt 1800
   ttaacgatca gttcgccgat gcagatattc gtaattatgc gggcaacgtc tggtatcagc 1860
   gcgaagtctt tataccgaaa ggttgggcag gccagcgtat cgtgctgcgt ttcgatgcgg 1920
   tcactcatta cggcaaagtg tgggtcaata atcaggaagt gatggagcat cagggcggct 1980
   atacgccatt tgaagccgat gtcacgccgt atgttattgc cgggaaaagt gtacgtatca 2040
   ccgtttgtgt gaacaacgaa ctgaactggc agactatccc gccgggaatg gtgattaccg 2100
   acgaaaacgg caagaaaaag cagtcttact tccatgattt ctttaactat gccggaatcc 2160
   atcgcagcgt aatgctctac accacgccga acacctgggt ggacgatatc accgtggtga 2220
   cgcatgtcgc gcaagactgt aaccacgcgt ctgttgactg gcaggtggtg gccaatggtg 2280
   atgtcagcgt tgaactgcgt gatgcggatc aacaggtggt tgcaactgga caaggcacta 2340
   gcgggacttt gcaagtggtg aatccgcacc tctggcaacc gggtgaaggt tatctctatg 2400
   aactgtgcgt cacagccaaa agccagacag agtgtgatat ctacccgctt cgcgtcggca 2460
   tccggtcagt ggcagtgaag ggccaacagt tcctgattaa ccacaaaccg ttctacttta 2520
   ctggctttgg tcgtcatgaa gatgcggact tacgtggcaa aggattcgat aacgtgctga 2580
   tggtgcacga ccacgcatta atggactgga ttggggccaa ctcctaccgt acctcgcatt 2640
   acccttacgc tgaagagatg ctcgactggg cagatgaaca tggcatcgtg gtgattgatg 2700
   aaactgctgc tgtcggcttt aacctctctt taggcattgg tttcgaagcg ggcaacaagc 2760
   cgaaagaact gtacagcgaa gaggcagtca acggggaaac tcagcaagcg cacttacagg 2820
   cgattaaaga gctgatagcg cgtgacaaaa accacccaag cgtggtgatg tggagtattg 2880
   ccaacgaacc ggatacccgt ccgcaagtgc acgggaatat ttcgccactg gcggaagcaa 2940
   cgcgtaaact cgacccgacg cgtccgatca cctgcgtcaa tgtaatgttc tgcgacgctc 3000
   acaccgatac catcagcgat ctctttgatg tgctgtgcct gaaccgttat tacggatggt 3060
   atgtccaaag cggcgatttg gaaacggcag agaaggtact ggaaaaagaa cttctggcct 3120
   ggcaggagaa actgcatcag ccgattatca tcaccgaata cggcgtggat acgttagccg 3180
   ggctgcactc aatgtacacc gacatgtgga gtgaagagta tcagtgtgca tggctggata 3240
   tgtatcaccg cgtctttgat cgcgtcagcg ccgtcgtcgg tgaacaggta tggaatttcg 3300
   ccgattttgc gacctcgcaa ggcatattgc gcgttggcgg taacaagaaa gggatcttca 3360
   ctcgcgaccg caaaccgaag tcggcggctt ttctgctgca aaaacgctgg actggcatga 3420
   acttcggtga aaaaccgcag cagggaggca aacaatgaat caacaactct cctggcgcac 3480
   catcgtcggc tacagcctcg ggaattgcta ccnntctaga attcggctga aatcaccagt 3540
   ctctctctac aaatctatct ctctctattt tctccataaa taatgtgtga gtagtttccc 3600
   gataagggaa attagggttc ttatagggtt tcgctcatgt gttgagcata taagaaaccc 3660
   ttagtatgta tttgtatttg taaaatactt ctatcaataa aatttctaat tcctaaaacc 3720
   aaaatccagt actaaaatcc agatctccta aagtccctat agatctttgt cgtgaatata 3780
   aaccagacac gagacgacta aacctggagc ccagacgccg ttcgaagcta gaagtaccgc 3840
   ttaggcagga ggccgttagg gaaaagatgc taaggcaggg ttggttacgt tgactccccc 3900
   gtaggtttgg tttaaatatg atgaagtgga cggaaggaag gaggaagaca aggaaggata 3960
   aggttgcagg ccctgtgcaa ggtaagaaga tggaaatttg atagaggtac gctactatac 4020
   ttatactata cgctaaggga atgcttgtat ttatacccta taccccctaa taacccctta 4080
   tcaatttaag aaataatccg cataagcccc cgcttaaaaa ttggtatcag agccatgaat 4140
   aggtctatga ccaaaactca agaggataaa acctcaccaa aatacgaaag agttcttaac 4200
   tctaaagata aaagatcttt caagatcaaa actagttccc tcacaccggt gacggggat 4259
<210> 8
   <211> 4448
   <212> DNA
   <213> artificial
<220>
   <223> proTaHMWG - intAtFAD2 - EcGUSintStLS1 - terVirCaMV
<220>
   <221> promoter
   <222> (1).. 418)
   <223> TaHMWG promoter
<220>
   <221> Intron
   <222> (467) .. (1601)
   <223> FAD 2 intron
<220>
   <221> Coding_sequence
   <222> (1638) .. (3632)
   <223> GUS protein
<220>
   <221> terminator
   <222> (3711)..(4498)
   <223> CAMV terminator
<400> 8
   agctttgagt ggccgtagat ttgcaaaagc aatggctaac agacacatat tctgccaaac 60
   cccaagaagg ataatcactt ttcttagata aaaaagaaca gaccaatata caaacatcca 120
   cacttctgca aacaatacat cagaactagg attacgccga ttacgtggct ttagcagact 180
   gtccaaaaat ctgttttgca aagctccaat tgctccttgc ttatccagct tcttttgtgt 240
   tggcaaactg cgcttttcca accgattttg ttcttctcgc gctttcttct taggctaaac 300
   aaacctcacc gtgcacgcag ccatggtcct gaaccttcac ctcgtcccta taaaagccta 360
   gccaaccttc acaatcttat catcacccac aacaccgagc accacaaact agagatccga 420
   attaattctc gcggccgctc tagaactagt ggatcccccg ggccaggtcc gtcgcttctc 480
   ttccatttct tctcattttc gattttgatt cttatttctt tccagtagct cctgctctgt 540
   gaatttctcc gctcacgata gatctgctta tactccttac attcaacctt agatctggtc 600
   tcgattctct gtttctctgt ttttttcttt tggtcgagaa tctgatgttt gtttatgttc 660
   tgtcaccatt aataataatg aactctctca ttcatacaat gattagtttc tctcgtctac 720
   aaaacgatat gttgcatttt cacttttctt cttttttctt aagatgattt gctttgacca 780
   atttgtttag atctttattc tattttattt tctggtgggt tggtggaaat tgaaaaaaaa 840
   aaaaacagca taaattgtta tttgttaatg tattcatttt ttggctattt gttctgggta 900
   aaaatctgct tctactattg aatctttcct ggatttttta ctcctattgg gtttttatag 960
   taaaaataca taataaaagg aaaacaaaag ttttatagat tctcttaaac cccttacgat 1020
   aaaagttgga atcaaaataa ttcaggatca gatgctcttt gattgattca gatgcgatta 1080
   cagttgcatg gcaaattttc tagatccgtc gtcacatttt attttctgtt taaatatcta 1140
   aatctgatat atgatgtcga tcgacaaatt ctggtggctt atacatcact tcaactgttt 1200
   tcttttggct ttgtttgtca acttggtttt caatacgatt tgtgatttcg atcgctgaat 1260
   ttttaataca agcaaactga tgttaaccac aagcaagaga tgtgacctgc cttattaaca 1320
   tcgtattact tactactagt cgtattctca acgcaatcgt ttttgtattt ctcacattat 1380
   gccgcttctc tactctttat tccttttggt ccacgcattt tctatttgtg gcaatccctt 1440
   tcacaacctg atttcccact ttggatcatt tgtctgaaga ctctcttgaa tcgttaccac 1500
   ttgtttcttg tgcatgctct gttttttaga attaatgata aaactattcc atagtcttga 1560
   gttttcagct tgttgattct tttgcttttg gttttctgca gaaacccggg ctgcaggaat 1620
   tcgatatcaa gcttcccatg gtacgtcctg tagaaacccc aacccgtgaa atcaaaaaac 1680
   tcgacggcct gtgggcattc agtctggatc gcgaaaactg tggaattgat cagcgttggt 1740
   gggaaagcgc gttacaagaa agccgggcaa ttgctgtgcc aggcagtttt aacgatcagt 1800
   tcgccgatgc agatattcgt aattatgcgg gcaacgtctg gtatcagcgc gaagtcttta 1860
   taccgaaagg ttgggcaggc cagcgtatcg tgctgcgttt cgatgcggtc actcattacg 1920
   gcaaagtgtg ggtcaataat caggaagtga tggagcatca gggcggctat acgccatttg 1980
   aagccgatgt cacgccgtat gttattgccg ggaaaagtgt acgtaagttt ctgcttctac 2040
   ctttgatata tatataataa ttatcattaa ttagtagtaa tataatattt caaatatttt 2100
   tttcaaaata aaagaatgta gtatatagca attgcttttc tgtagtttat aagtgtgtat 2160
   attttaattt ataacttttc taatatatga ccaaaatttg ttgatgtgca ggtatcaccg 2220
   tttgtgtgaa caacgaactg aactggcaga ctatcccgcc gggaatggtg attaccgacg 2280
   aaaacggcaa gaaaaagcag tcttacttcc atgatttctt taactatgcc ggaatccatc 2340
   gcagcgtaat gctctacacc acgccgaaca cctgggtgga cgatatcacc gtggtgacgc 2400
   atgtcgcgca agactgtaac cacgcgtctg ttgactggca ggtggtggcc aatggtgatg 2460
   tcagcgttga actgcgtgat gcggatcaac aggtggttgc aactggacaa ggcactagcg 2520
   ggactttgca agtggtgaat ccgcacctct ggcaaccggg tgaaggttat ctctatgaac 2580
   tgtgcgtcac agccaaaagc cagacagagt gtgatatcta cccgcttcgc gtcggcatcc 2640
   ggtcagtggc agtgaagggc caacagttcc tgattaacca caaaccgttc tactttactg 2700
   gctttggtcg tcatgaagat gcggacttac gtggcaaagg attcgataac gtgctgatgg 2760
   tgcacgacca cgcattaatg gactggattg gggccaactc ctaccgtacc tcgcattacc 2820
   cttacgctga agagatgctc gactgggcag atgaacatgg catcgtggtg attgatgaaa 2880
   ctgctgctgt cggctttaac ctctctttag gcattggttt cgaagcgggc aacaagccga   2940
   aagaactgta cagcgaagag gcagtcaacg gggaaactca gcaagcgcac ttacaggcga   3000
   ttaaagagct gatagcgcgt gacaaaaacc acccaagcgt ggtgatgtgg agtattgcca   3060
   acgaaccgga tacccgtccg caagtgcacg ggaatatttc gccactggcg gaagcaacgc   3120
   gtaaactcga cccgacgcgt ccgatcacct gcgtcaatgt aatgttctgc gacgctcaca   3180
   ccgataccat cagcgatctc tttgatgtgc tgtgcctgaa ccgttattac ggatggtatg   3240
   tccaaagcgg cgatttggaa acggcagaga aggtactgga aaaagaactt ctggcctggc   3300
   aggagaaact gcatcagccg attatcatca ccgaatacgg cgtggatacg ttagccgggc   3360
   tgcactcaat gtacaccgac atgtggagtg aagagtatca gtgtgcatgg ctggatatgt   3420
   atcaccgcgt ctttgatcgc gtcagcgccg tcgtcggtga acaggtatgg aatttcgccg   3480
   attttgcgac ctcgcaaggc atattgcgcg ttggcggtaa caagaaaggg atcttcactc   3540
   gcgaccgcaa accgaagtcg gcggcttttc tgctgcaaaa acgctggact ggcatgaact   3600
   tcggtgaaaa accgcagcag ggaggcaaac aatgaatcaa caactctcct ggcgcaccat   3660
   cgtcggctac agcctcggtg acgtcggatc ccccgggctg caggaattcg gtacgctgaa   3720
   atcaccagtc tctctctaca aatctatctc tctctatttt ctccataaat aatgtgtgag   3780
   tagtttcccg ataagggaaa ttagggttct tatagggttt cgctcatgtg ttgagcatat   3840
   aagaaaccct tagtatgtat ttgtatttgt aaaatacttc tatcaataaa atttctaatt   3900
   cctaaaacca aaatccagta ctaaaatcca gatctcctaa agtccctata gatctttgtc   3960
   gtgaatataa accagacacg agacgactaa acctggagcc cagacgccgt tcgaagctag   4020
   aagtaccgct taggcaggag gccgttaggg aaaagatgct aaggcagggt tggttacgtt   4080
   gactcccccg taggtttggt ttaaatatga tgaagtggac ggaaggaagg aggaagacaa   4140
   ggaaggataa ggttgcaggc cctgtgcaag gtaagaagat ggaaatttga tagaggtacg   4200
   ctactatact tatactatac gctaagggaa tgcttgtatt tataccctat accccctaat   4260
   aaccccttat caatttaaga aataatccgc ataagccccc gcttaaaaat tggtatcaga   4320
   gccatgaata ggtctatgac caaaactcaa gaggataaaa cctcaccaaa atacgaaaga   4380
   gttcttaact ctaaagataa aagatctttc aagatcaaaa ctagttccct cacaccggtg   4440
   acggggat                                                            4448
<210> 9
   <211> 25
   <212> DNA
   <213> Artificial
<220>
   <223> forward primer
<400> 9
   gggccaggtc cgtcgcttct cttcc 25
<210> 10
   <211> 24
   <212> DNA
   <213> artificial
<220>
   <223> reverse primer
<400> 10
   gggtttctgc agaaaaccaa aagc 24
<210> 11
   <211> 19
   <212> DNA
   <213> artificial
<220>
   <223> forward primer
<400> 11
   gatgctctag atctgctgg 19
<210> 12
   <211> 29
   <212> DNA
   <213> artificial
<220>
   <223> reverse primer
<400> 12
   ggccatggct tgctctgata ccagacggg 29

## Description

### BACKGROUND OF THE INVENTION

Efficient transformation systems are available in plants, in particular in monocot species especially those of economic importance such as maize, wheat, barley and rice. It is now possible to contemplate the introduction of a range of traits by such technique. The most widely used techniques are the delivery of naked DNA by micro projectile bombardment or the use of Agrobacterium as a vector. In both cases the gene of interest and associated sequences are introduced into a suitable target plant tissue and become stably integrated into the plant genome. The target tissue has the potential to regenerate into a whole, fully-fertile plant.

In order for the introduced gene to be expressed, it must be driven by a promoter. Promoters can be of different types; constitutive or tissue and temporally specific. Widely use promoters include those derived from virus and bacteria; including Cauliflower Mosaic Virus 35S and octopine and nopaline synthase and RuBISCo, actin and ubiquitin from plant species.

The most widely used promoters are constitutive because they are generally used to control the selectable marker gene and can also be used to drive the gene of interest.

Although suitable promoters for monocot species are known, insufficient numbers are available for use in biotechnology applications. Transformation may require that one promoter drives the selectable marker and a separate promoter drives the gene of interest.

It is indeed undesirable to use the same promoter for both genes for several reasons. Initially the cloning and construct preparation becomes problematical. Sequence duplications can lead to gene deletions and other corruptions which would result in elimination of gene expression. Sequence duplication subsequently *in planta* can result in gene silencing. In a longer term consideration of breeding programmes, either gene stacking by retransformation or crossing of existing transgenic lines, the lack of choice of promoters will become increasingly limiting.

Strong promoters are preferred as the efficiency of production of useful transgenic plants is increased, in two ways. Firstly more plants are initially selected in tissue culture due to sufficient expression of the selectable marker to survive the selection conditions and secondly more plants thus selected will have a good phenotype resulting from the expression of the gene or genes of interest. In order to increase the number of suitable promoters it was discovered that the strength of a promoter can be increased by use of an intron.

Introns interrupt the coding regions of Eukaryotic genes and are removed post-transcription by the process of splicing. The expression of genes in animal cells has been known to be intron dependant since the late 1970s but the first demonstration of the effect of the inclusion of an intron in a plant species was not until 1987 (Callis et al , Genes and Development 1: 1183-1200).

The most widely used introns are the first introns of rice Actin and maize Ubiquitin, used in combination with the natural promoters. These promoters were the first strong promoters identified for monocot species. Rice actin was initially identified as a suitable candidate gene for provision of a strong constitutive promoter because actin is a fundamental and essential component of the eukaryotic cell and cytoskeleton. A rice actin gene was identified that encoded a transcript that was abundant in all rice tissues at all developmental stages (McElroy et al 1990, Plant Cell 2:163.) Further characterisation revealed that the presence of the first intron was essential for the efficient function of the promoter *per se.* Similarly the maize ubiquitin promoter was identified as having a high level constitutive activity dependant on the presence of its own first intron (Morris et al 1993, Plant Molecular Biology 21: 895 - 906).

The enhancing effect of the rice actin first intron and the maize ubiquitin first intron can also be combined with heterologous promoter sequences which alone have minimal activity in monocot species. (Vain et al , 1996, Plant Cell reports 15: 489 - 494)

A limited number of plant introns have been identified that can be combined with suboptimal heterologous promoters to create novel promoters sufficiently strong to use for biotechnological applications. Early comparisons of a range of introns revealed that not all have a significantly enhancing activity in combination with a suboptimal promoter. (Vain et al, 1996)

Nevertheless, intron duplication is undesirable for same reasons that promoter duplication should be avoided.

There is therefore a continuing need to identify new suitable introns to enhance expression in plants, and especially in monocot transformation systems. These introns need to be able to increase the level of expression when combined with various promoters.

At the present time there are very few such introns identified and this ultimately limits the number of genetic transformations that can be carried out in a given monocot species.

The invention now provides a new intron that is usable for increasing expression of an heterologous gene in a plant cell, and in particular a monocot plant cell, such as wheat, maize, barley, or rice.

### SUMMARY OF THE INVENTION

The inventors have indeed surprisingly observed that presence of the first intron of the FAD2 gene of Arabidopsis, which was believed to have no effect on expression, can effectively enhance the efficiency of a promoter.

The FAD2 gene is the gene coding for the microsomal omega-6 fatty acid desaturase (FAD2/delta-12 desaturase), and its sequence is well known by the person skilled in the art, with the availability of the sequence of the chromosome 3 *Arabidopsis thaliana* genome.

Other FAD2 genes have been identified (for example *Brassica oleracea,* represented by AF181726 in GenBank, soybean with GenBank protein accession number P48628, *Brassica napus,* GenBank protein accession number P48627...). The identification of the first introns of FAD2 genes of other species is within the skill of the person in the art, by the differences between the cDNA and genomic sequences.

### FIGURES

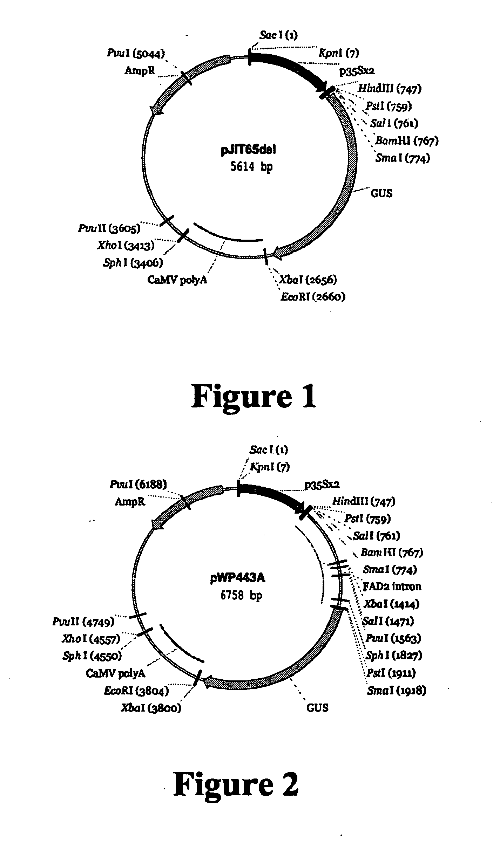

Figure 1: Schematic representation of pJIT65deI
Figure 2: Schematic representation of pWP443A

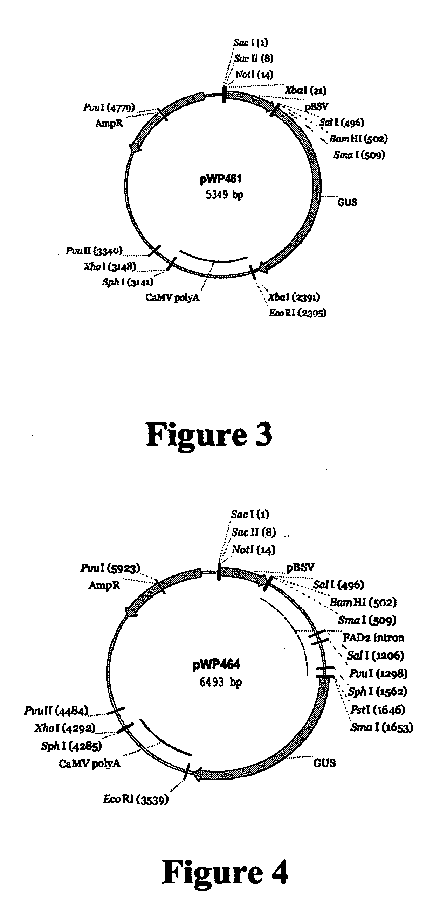

Figure 3 : Schematic representation of pWP461
Figure 4: Schematic representation of pWP464

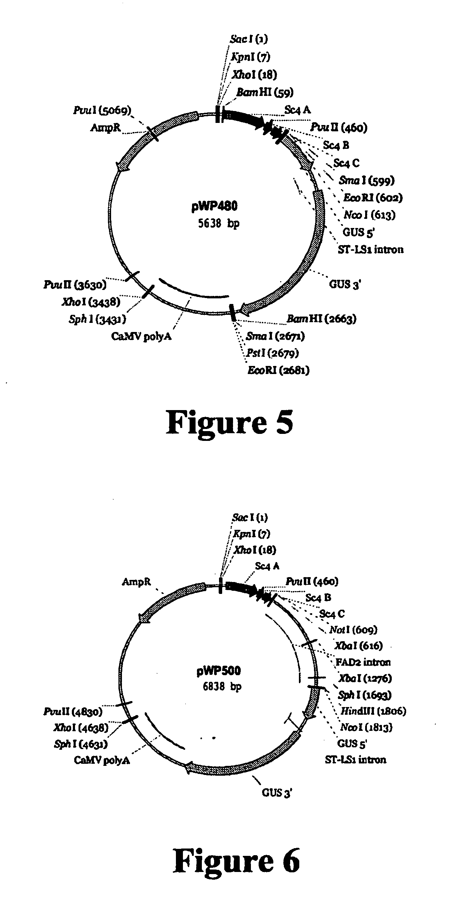

Figure 5: Schematic representation of pWP480
Figure 6 : Schematic representation of pWP500

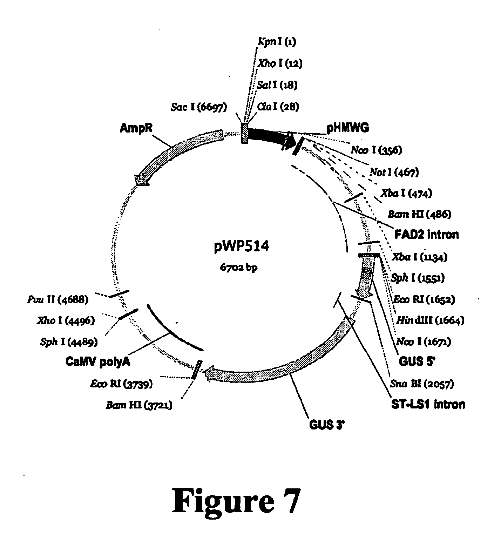

Figure 7 : Schematic representation of pWP514
Figure 8: Transient expression comparison in wheat. Embryos bombarded with 3 promoters with and without FAD2 intron.
Figure 9: Transient expression comparison in barley a) embryos bombarded with pWP480 b) embryos bombarded with pWP500
Figure 10: Leaves from wheat stably transformed wheat with a) pBSV without FAD2 intron, or b) pBSV with FAD2 intron.

### DETAILED DESCRIPTION OF THE INVENTION

The invention thus relates to a genetic construct for the expression of a polypeptide in a plant cell, comprising:
(a) a promoter operative within said plant cell,
(b) the first intron of a FAD2 gene as specified below,
(c) a nucleotide sequence operably linked to said promoter, wherein said nucleotide sequence encodes said polypeptide,
(d) a polyadenylation site (PAA) operably linked to said nucleotide sequence.

Said polynucleotide is called "polynucleotide of interest" and is coded by a "gene of interest".

A "promoter operative in a plant cell" is intended to mean a sequence that directs the initiation of transcription in a plant cell. It can derive from one or multiple sources, be natural or synthetic.

In the preferred embodiment, the genetic construct of the invention is such that said FAD2 gene is from *Arabidopsis thaliana* (GenBank AJ271841), wherein said first intron is represented by SEQ ID N° 1, also indicated in SEQ ID N° 3, 5, 7 and 8.

In the context of the present invention, "all or part of the first intron of a FAD2 gene" is to be considered as the full sequence of the first intron of a FAD 2 gene, or a fragment comprising at least 200, 500 or 1000 nucleotides of said sequence.

The promoter could be any promoter, as described above. In a preferred embodiment, said promoter is a tissue-specific promoter. In another embodiment, said promoter is a constitutive promoter. Thus it is clear that the envisaged promoter is a heterologous promoter, *i.e*. different of the natural FAD2 promoter.

promoter sequences of genes which are expressed naturally in plants can be of plant, bacterial or viral origin. Suitable constitutive promoters include but are not restricted to octopine synthase, nopaline synthase, mannopine synthase derived from the T-DNA of *Agrobacterium tumefaciens;* CaMV35S and CaMV19S from Cauliflower Mosaic Virus; rice actin, maize ubiquitin and histone promoters from plant species.

Tissue specific promoters include but are not restricted to rolC from *Agrobacterium rhizogenes;* RTBV from Rice Tungro Bacilliform Virus; LMW and HMW glutenin from wheat; alcohol dehydrogenase, waxy, zein from maize and, AoPR1 from Asparagus.

All these promoters (and others) are well described in the art.

Whilst the most widely used promoters are constitutive or tissue specific it is also possible to contemplate the use of inducible promoters such as PinII or AoPRT-L (WO 99/66057); more specifically light inducible including RUBISCO small subunit and chlorophyll a/b binding protein from a range of species and synthetic promoters eg EMU (US19900525866).

The genetic construct according to the invention is introduced within the plant cell on a vector. In an embodiment, said vector is maintained as an episomal vector within the plant cell. In the preferred embodiment, said vector is capable of integration within the genome of said plant cell. Integration of a genetic construct within a plant cell is performed using methods known by those skilled in the art, for example transformation by Agrobacterium, or gun bombardment.

Agrobacterium tumefaciens has been widely and efficiently used to transform numerous species dicots and including monocots such as maize, rice, barley and wheat (WO 00/63398). Alternative gene transfer and transformation methods include protoplast transformation through calcium, polyethylene glycol or electroporation mediated uptake of naked DNA. Additional methods include introduction of DNA into intact cells or regenerable tissues by microinjection, silicon carbide fibres or, most widely, microprojectile bombardment. All these methods are now well known in the art.

The invention also relates to a method for enhancing the expression of a polypeptide in a plant cell, comprising the step of introducing the genetic construct of the invention within said plant cell, for example using the methods as mentioned above. Expression is enhanced as compared to expression obtained when the FAD2 intron is not present within the construct.

Expression relates to transcription and translation of a gene so that a protein is made.

In another aspect, the invention relates to a method for increasing the expression rate of a gene, under the control of a promoter in a plant cell, said method comprising the steps of:
(a) inserting the first intron of the FAD 2 gene as defined above between said promoter and the translation start of said gene, thereby forming an intron-modified gene; and
(b) introducing said intron-modified gene into a plant cell such that said gene is expressed under the control of said intron in said plant cell, whereby the expression rate of said gene is increased as compared to an unmodified gene.

Said gene is called "gene of interest".

In particular, said gene can be a selectable marker. These selectable markers include, but are not limited to, antibiotic resistance genes, herbicide resistance genes or visible marker genes. Other phenotypic markers are known in the art and may be used in this invention.

A number of selective agents and resistance genes are known in the art. (See, for example, Hauptmann et al., 1988 ; Dekeyser et al., 1988 ; Eichholtz et al., 1987 ; and Meijer et al., 19991).

Notably the selectable marker used can be the bar gene conferring resistance to bialaphos (White et al., 1990), the sulfonamide herbicide Asulam resistance gene, sul (described in WO 98/49316) encoding a type I dihydropterate synthase (DHPS), the nptII gene conferring resistance to a group of antibiotics including kanamycin, G418, paromomycin and neomycin (Bevan et al., 1983), the hph gene conferring resistance to hygromycin (Gritz et al., 1983), the EPSPS gene conferring tolerance to glyphosate (US 5,188,642), the HPPD gene conferring resistance to isoxazoles (WO 96/38567), the gene encoding for the GUS enzyme, the green fluorescent protein (GFP), expression of which, confers a recognisible physical characteristic to transformed cells, the chloramphenicol transferase gene, expression of which, detoxifies chloramphenicol.

In another embodiment, said gene encodes a protein to impart insect resistance, more preferably genes which encode for Bacillus thuringiensis (Bt) endotoxins (inter alia, U.S. Patent Nos. 5,460,963; 5,683,691; 5,545,565; 5,530,197; 5,317,096). The nucleic acids that are preferably embraced by the instant invention are cryI, cryII, cryIII, and cryIV genes. More preferably, the genes include: cryIA(a), cryIA(b); cryIA(c); and cryIIIA(a). Most preferably the gene is cryIA(a), cryIA(b) or cryIA(c).

Other genes of interest are
- the bacterial gene dapA for increasing the level of lysine;
- the gene for endotoxin Bt or for a protease inhibitor or for proteins extracted from bacteria such as Photorabus (WO 97/17432 & WO 98/08932), for resistance to insects;
- among the proteins or peptides of interest which confer novel properties of resistance to diseases, mention will be made in particular of chitinases (WO 92/01792), glucanases (WO 93/02197), oxalate oxidase (WO 94/13790) or antibacterial and/or antifungal peptides, in particular the peptides of less than 100 amino acids rich in cysteins, such as plant thionins or defensins, and more particularly lytic peptides of any origins comprising one or more disulfide bridges between the cysteins and regions comprising basic amino acids, in particular the following lytic peptides: androctonin (WO 97/30082 and WO 99/09189), drosomicin (WO 99/02717), thanatin (WO 99/24594) or heliomycin (WO 99/53053). According to a particular embodiment of the invention, the protein or peptide of interest is chosen from fungal elicitor peptides, in particular elicitins (Kamoun et al., 1993; Panabières et al., 1995).
- genes involved in the biosynthetic processes which lead to a change in the quality of the products of the transgenic plant, such as the genes encoding enzymes for the biosynthesis or degradation of starch (i.e. synthases, starch-branching enzymes, etc.); genes encoding grain storage proteins (i.e. subunits of glutenins, gliadins, hordeins); genes related to the strength of the grain in wheat (i.e. puroindolines).
- genes which modify the constitution of the modified plants, in particular the content and the quality of certain essential fatty acids (EP 666 918) or the content and the quality of the proteins, in particular in the leaves and/or the grains of said plants. Mention will be made, in particular, of the genes encoding proteins enriched in sulfur-containing amino acids (Korit, A.A. et al.; WO 98/20133; WO 97/41239; WO 95/31554; WO 94/20828; WO 92/14822). The function of these proteins enriched in sulfur-containing amino acids will also be to trap and store excess cystein and/or methionine, making it possible to avoid the possible problems of toxicity linked to an overproduction of these sulfur-containing amino acids by trapping them. Mention may also be made of genes encoding peptides rich in sulfur-containing amino acids, and more particularly in cysteins, said peptides also having antibacterial and/or antifungal activity. Mention will be made more particularly of plant defensins, and also lytic peptides of any origin, and more particularly the following lytic peptides: androctonin (WO 97/30082 and WO 99/09189), drosomicin (WO 99/02717), thanatin (WO 99/24594) or heliomycin (WO 99/53053).
- genes for artificial male sterility (i.e. barnase, and PR-glucanase under the control of a suitable promoter) may also be used for the production of hybrid seeds.

In another aspect, the invention relates to the use of the first intron of the FAD2 gene as defined above for enhancing expression of a polypeptide in a plant cell.

In another aspect, the FAD2 first intron as defined above may be used to convert a tissue specific promoter into a constitutive promoter. For example, the strong seed specific HMW glutenin promoter is converted into a strong/ useful promoter active in many tissue types, when used with the FAD 2 first intron.

### EXAMPLES

All DNA modifications and digestions were performed using enzymes according to the manufacturers' instruction and following protocols described in Sambrook and Russell, 2001; Molecular Cloning, A Laboratory Manual.

### Example 1. Cloning of FAD 2 intron

The first intron of the FAD 2 gene was obtained by the polymerase chain reaction (PCR) from *Arabidopsis thaliana* genomic DNA. The following pair of primers were used:

| | |
|---|---|
| GGGCCAGGTCCGTCGCTTCTCTTCC | FADfor (SEQ ID N° 9) |
| GGGTTTCTGCAGAAAACCAAAAGC | FADrev (SEQ ID N° 10) |

A standard PCR reaction was carried out, with amplification under the following conditions: 30 cycles of 97°C for 30 seconds, 57°C for 30 seconds and 74°C for 1 minute 40 seconds. The reaction products were separated by agarose gel electrophoresis.

The desired 1146 bp product was excised from the gel and ligated into the SmaI restriction site of pTZ18 (standard cloning vector; Pharmacia) to create pWP430. The fidelity of the clone was confirmed by sequencing.

### Example 2. Preparation of GUS constructs

Several promoters, which are known in the field of plant biotechnology were identified, to be tested with and without the FAD2 intron. These included but were not limited to Cauliflower Mosaic Virus 35S (Odell et al,Nature. 1985 Feb 28-Mar 6;313(6005):810-2), Banana Streak Virus promoter of ORF1 (WO 99/43836), Sc4, a member of the Plant Expression (PLEX) promoter family (EP 785999; US 6211431) and HMW glutenin (HMWG) promoter. Constructs were prepared from well-characterised genetic elements and cloned into widely used plasmid vectors.

### 35S. pJIT65del

The 35S promoter was cloned in a pUC vector, driving the GUS gene (Jefferson et al, PNAS 83: 8447-8451, 1986), to obtain plasmid pJIT65. This plasmid was modified by digestion with *Xba*I and *EcoR*I to removing intervening *BamH*I and *Sma*I sites. This modified version was named as pJIT65del.

### 35S + FAD2 intron: pWP443A

The FAD2 intron was inserted between the 35S promoter and the GUS gene, specifically into the 5'UTR, by digesting pWP430 with *Sma*I and ligating the intron into *Sma*I digested pJIT65del, to create pWP443A.

### BSV: pWP461

The BSV promoter was cloned into the *EcoRV* site of Bluescript KS to create pWP453B. The following primers were used:

| | |
|---|---|
| GATGCTCTAGATCTGCTGG | BSVFor (SEQ ID N° 11) |
| GGCCATGGCTTGCTCTGATACCAGACGGG | BSVRev (SEQ ID N° 12) |

pWP453B was digested with *SstI* and *Sal*I to isolate the BSV promoter fragment which was then ligated into *Sst*I and *Sal*I digested JTr65del, thus replacing the 35S promoter to create pWP461.

### BSV + FAD2 intron:pWP464

To create the combination of the BSV promoter with the FAD2 intron the 35S promoter of pWP443A was replaced by the BSV promoter from pWP461. Both plasmids were digested with SstI and BamHI and fragments ligated to produce pWP464.

### Sc4: pWP480

A previously prepared construct, pKH2, having the Sc4 promoter driving the GUS gene, with the inclusion of the rice actin first intron in the 5'UTR, was digested with SstII and Ncol to remove the actin intron and create pWP480.

### Sc4 +FAD2 intron:pWP 500

Similarly to the creation of pWP464, to combine the Sc4 promoter with the FAD2 intron the 35S promoter of pWP443A was replaced by the Sc4 promoter from pWP480 using suitable digests.

### HMWG + intron: pWP514

A pUC clone of the HMWG promoter driving the GUS gene (Glu-1Dx5-GUS2) was obtained as described in Halford et al 1989 (Plant Science 62, 207-16). The FAD2 intron was inserted between the HMWG promoter and the GUS gene, specifically into the 5'UTR, to create pWP514.

### Example 3 Transient expression in wheat

### a) Initial assessment of functioning of constructs

A range of plant tissues and calli were bombarded with particles coated with one of the four promoter constructs including the FAD2 intron, according to methods known in the art. The tissues were immersed in a solution of the histochemical substrate. X-glucuronide, and a subjective assessment was made of the activity of the construct. In each case a significant number of blue spots were observed on each of the tissues tested.

| | TRANSIENT EXPRESSION |
|---|---|
| pWP443A | Strong expression on embryo, leaf and callus |
| pWP500 | Strong expression on embryo, leaf and callus |
| pWP464 | Strong expression on embryo, leaf and callus |
| pWP514 | Good expression on embryo, leaf and endosperms |

It is especially worthy of note that there was significant expression in leaf and embryo tissues following bombardment with the HMWG construct (pWP514). The HMWG promoter is naturally strong endosperm-specific promoter. The inclusion of the FAD2 intron has converted it into a strong constitutive promoter.

### b) Comparison of constitutive promoters with and without introns

Having established that the promoters modified by the inclusion of the FAD2 intron were functioning a quantitative comparison was made between the promoters with and without the intron.

Isolated embryos were arranged in 4x4 arrays. Thirty-two embryos were bombarded with each construct.

Co-bombardment with a second construct containing the Green Fluorescent Protein (GFP) under control of the rice actin promoter, was performed. Expression of GFP can be non-destructively tested and hence the relative efficiency of each individual bombardment can be normalized to allow comparison between individual bombardments.

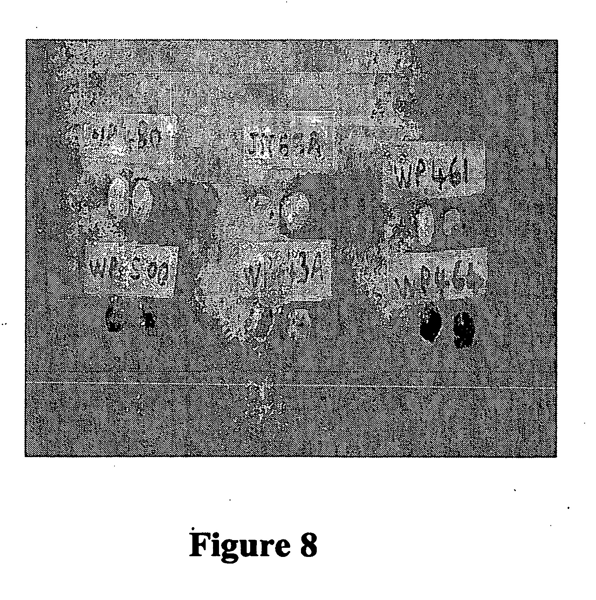

The level of expression for each construct was again visually assessed by histochemical assay for the GUS gene (figure 8) which enabled both a quantitative (counting the number of blue foci by 4x4 array), and a qualitative assessment (consideration of the size of blue foci).

The increase in expression due to inclusion of the FAD2 intron was 5.6 fold for the Sc4 promoter, 8 fold for the double 35S promoter and most remarkably 161 fold for the BSV promoter. Thus the expression of the weakest promoter (BSV) is increased to a level comparable with the maize ubiquitin promoter (pAAA) which is widely used because it is known to be very strong.

| | pSc4 | | | | pBSV | | | |
|---|---|---|---|---|---|---|---|---|
| | pWP480 | | pWP500 | | pWP461 | | pWP464 | |
| GFP | 1648 | 698 | 1501 | 1945 | 989 | 1015 | 1504 | 1384 |
| GUS | 610 | 142 | 2340 | 3101 | 36 | 14 | 5067 | 4287 |

| Note on GUS spots | Very small spots, difficult to see with the naked eye | | Big spots, well seen but no spreading nor covering | | Very small spots (two big spots on two different embryos) | | Very big, 'spreading' spots | |
|---|---|---|---|---|---|---|---|---|
| Ratio GUS/GFP | 0.37 | 0.20 | 1.55 | 1.59 | 0.03 | 0.01 | 3.36 | 3.09 |
| Average | 0.28 | | 1.57 | | 0.02 | | 3.22 | |
| Ratio with/without | 5.6 | | | | 161 | | | |

| | p35S | | | | Positive Control | |
|---|---|---|---|---|---|---|
| | pJIT65del | | pWP443A | | pAAA | |
| GFP | 470 | 922 | 711 | 687 | 1502 | 1390 |
| GUS | 93 | 296 | 1539 | 1279 | 7200 | 7200 |
| Note on GUS spots | Very small dots, sometimes not seen with the naked eye, few big ones | | Plenty of rather big spots | | Very big spots, 'spreading' all over the embryo difficult to score | |
| Ratio GUS/GFP | 0.19 | 0.32 | 2.16 | 1.86 | 4.79 | 5.17 |
| Average | 0.25 | | 2.01 | | 4.98 | |
| Ratio with/without | 8 | | | | / | |

A negative control with only gold particles yielded no spots.

### Example 4: Transient expression in barley

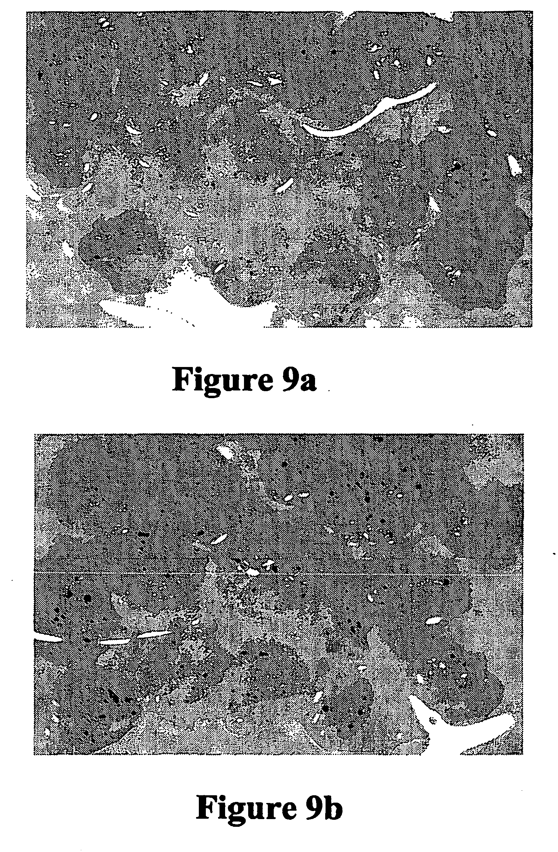

Other bombardment experiments were performed on barley embryos. The results also demonstrated that the presence of the FAD2 intron increases expression of the GUS protein in this species (figure 9). 2 plates of 16 embryos were bombarded with either pWP480 or pWP500 plasmid. The number of blue foci was scored per embryo and averaged.

Expression of co-bombarded GFP was again used to assess the relative efficiency of each individual bombardment and used to normalize and allow comparison between individual bombardments.

| | pWP480 | pWP500 |
|---|---|---|
| GFP | 63.9 | 60.7 |
| GUS | 27.00 | 92.2 |
| RatioGUS/GFP | 0.42 | 1.52 |
| Ratio +/- intron | 3.61 | |

### Example 5: Stable expression in wheat

Wheat embryos were stably transformed by bombardment (WO 98/49316). The embryos were bombarded with pWP461 or pWP464.

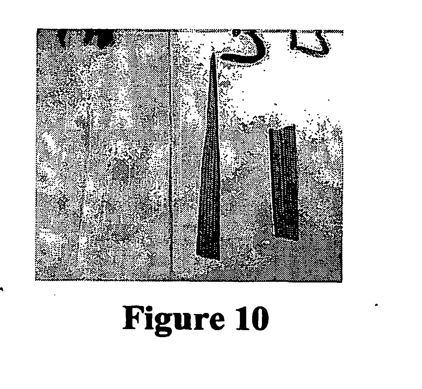

Transgenic plants were regenerated and grown, and expression of the GUS protein was studied by X-Gluc staining for glucuronidase activity. Following bombardment with WP461, no GUS expression was observed in regenerated transgenic plants. Following bombardment with WP464, strong constitutive expression could be observed in a range of tissues including leaf (Figure 10), root, male and female floral parts and seed.

### Example 6: Stable transformation of wheat

A selectable marker gene was prepared consisting of the Sc4 promoter, FAD 2 intron, the nptII coding region and nopaline synthase terminator. A comparable selectable marker gene was prepared consisting of the more widely used rice actin promoter and the associated first intron, the nptII coding region and nopaline synthase terminator. Both versions of the selectable marker gene were cloned into a suitable vector for wheat transformation. Wheat embryos were stably transformed by the Agrobacterium Seed Inoculation Method (SIM; WO 00/63398) using either

| Rice actin promoter + intron | Number of embryos | Transgenic events | Efficiency (%) |
|---|---|---|---|
| Experi ment 6 | 192 | 1 | 0.5 |
| Experi ment 7 | 206 | 19 | 9.2 |
| Experiment 8 | 220 | 3 | 1.4 |
| Experiment 9 | 225 | 4 | 1.8 |
| Experiment 10 | 145 | 1 | 0.7 |
| Experiment 11 | 139 | 18 | 13 |
| Total | 1127 | 46 | 4.1 |

version of the selectable marker, and stably transformed transgenic plantlets selected on plant tissue culture media containing a suitable concentration of Geneticin G418. The number of independent transgenic events was recorded and the transformation efficiency calculated as the percentage of treated embryos regenerating transgenic events.

| Sc4 promoter + FAD 2 intron | Number of embryos | Transgenic events | Efficiency (%) |
|---|---|---|---|
| Experiment 1 | 265 | 11 | 4.2 |
| Experiment 2 | 230 | 1 | 0.4 |
| Experiment 3 | 270 | 8 | 3.0 |
| Experiment 4 | 224 | 8 | 3.6 |
| Experiment 5 | 230 | 19 | 8.3 |
| Total | 1219 | 47 | 3.9 |

The rice actin promoter including the first intron is a very strong promoter widely used to drive the selectable marker in monocot species. Transformation frequencies were observed between 0.5 and 9.2 % with an average of 4.1%. When the selectable marker was driven by the Sc4 promoter in combination with the FAD 2 intron, transformation frequencies of 0.4 to 8.3%, with an average of 3.9% were observed.

Surprisingly the combination of the very weak Sc4 promoter with the FAD2 intron is as efficient as the widely used rice actin promoter when used to drive the selectable marker needed for stable wheat transformation.

### Example 7: Stable transformation of maize

Both versions of the selectable marker gene described in Example 6 were cloned into a suitable vector for maize transformation. Maize embryos were stably transformed essentially by the method of Ishida et al (1996) using either version of the selectable marker, and stably transformed transgenic plantlets selected on plant tissue culture media containing a suitable concentration of kanamycin. The number of independent transgenic events was recorded and the transformation efficiency calculated as the percentage of treated embryos regenerating transgenic events.

| Sc4 promoter + FAD 2 intron | Number of embryos | Transgenic events | Efficiency % |
|---|---|---|---|
| Experiment 1 | 2656 | 62 | 2.33 |
| 2 | 1455 | 12 | 0.82 |
| 3 | 1196 | 21 | 1.76 |
| 4 | 2212 | 30 | 1.36 |
| 5 | 130b | 8 | 0.61 |
| 6 | 1363 | 24 | 1.76 |
| 7 | 1247 | 24 | 1.92 |
| 8 | 1307 | 17 | 1.30 |
| 9 | 2297 | 18 | 0.78 |
| 10 | 2177 | 20 | 0.92 |
| 11 | 4306 | 67 | 1.56 |
| 12 | 2016 | 23 | 1.14 |
| 13 | 1218 | 6 | 0.49 |
| 14 | 1529 | 9 | 0.59 |
| Total | 26285 | 341 | 1.30 |

| Rice actin promoter + intron | Number of embryos | Transgenic events | Efficiency % |
|---|---|---|---|
| Experiment 1 | 1177 | 4 | 0.34 |
| 2 | 870 | 6 | 0.69 |
| 3 | 900 | 3 | 0.33 |
| 4 | 685 | 4 | 0.58 |
| 5 | 730 | 1 | 0.14 |
| 6 | 698 | 1 | 0.14 |
| 7 | 2349 | 7 | 0.30 |
| 8 | 1895 | 23 | 1.21 |
| 9 | 2515 | 42 | 1.67 |
| 10 | 1863 | 17 | 0.91 |
| 11 | 1448 | 10 | 0.69 |
| 12 | 2231 | 9 | 0.40 |
| 13 | 2170 | 20 | 0.92 |
| 14 | 3067 | 30 | 0.98 |
| 15 | 1135 | 4 | 0.35 |
| 16 | 2287 | 5 | 0.22 |
| 17 | 4132 | 54 | 1.31 |
| 18 | 1218 | 6 | 0.49 |
| 19 | 3250 | 12 | 0.37 |
| 20 | 2024 | 20 | 0.99 |
| 21 | 2013 | 20 | 0.99 |
| Total | 38657 | 298 | 0.77 |

Surprisingly the combination of the very weak Sc4 promoter with the FAD 2 intron is more efficient than the widely used rice actin promoter when used to drive the selectable marker needed for stable maize transformation (average 1.3% compared with 0.77%).

## Claims

1. A genetic construct for the expression of a polypeptide in a plant cell, comprising:
(a) a promoter operative within said plant cell,
(b) the first intron of the FAD2 gene, wherein said FAD2 gene is from Arabidopsis thaliana, and wherein said first intron is represented by SEQ ID N° 1
(c) a nucleotide sequence operably linked to said promoter, wherein said nucleotide sequence encodes said polypeptide,
(d) a polyadenylation site (PAA) operably linked to said nucleotide sequence
wherein said promoter is not a natural FAD2 promoter and wherein said intron is inserted between said promoter and the translation start of said nucleotide sequence.

2. The genetic construct of claim 1, wherein said promoter is a tissue-specific promoter.

3. The genetic construct of claim 1, wherein said promoter is a constitutive promoter.

4. The genetic construct of any of claims 1 to 3, capable of integration within the genome of said plant cell.

5. A method for enhancing the expression of a polypeptide in a plant cell, comprising the step of introducing the genetic construct of any of claims 1 to 4 within said plant cell.

6. A method for increasing, in a plant cell, the expression rate of a gene under the control of a promoter, said method comprising the steps of:
(a) inserting the first intron of the FAD 2 gene, wherein said FAD2 gene is from Arabidopsis thaliana, and wherein said first intron is represented by SEQ ID N° 1, between said promoter and the translation start of said gene, thereby forming an intron-modified gene; and
(b) introducing said intron-modified gene into a plant cell such that said gene is expressed under the control of said intron in said plant cell, whereby the expression rate of said gene is increased as compared to an unmodified gene
wherein said promoter is not a natural FAD2 promoter.

7. The use of the first intron of the FAD2 gene in combination with a promoter different of the natural FAD2 promoter for enhancing expression of a polypeptide in a plant cell, wherein said FAD2 gene is from Arabidopsis thaliana, and wherein said first intron is represented by SEQ ID N° 1.

8. A plant comprising a genetic construct according to claim 1 integrated in its genome.

9. The plant of claim 8, which is a monocot.

10. The plant of claim 9, which is chosen in the group consisting of wheat, barley, maize and rice.

## Patentansprüche

1. Genkonstrukt für die Expression eines Polypeptids in einer Pfanzenzelle, umfassend:
(a) einen innerhalb der Pflanzenzelle operativen Promoter,
(b) das erste Intron des FAD2-Gens, wobei das FAD2-Gen aus Arabidopsis thaliana stammt und wobei das erste Intron SEQ ID NO. 1 entspricht,
(c) eine mit dem Promoter operativ verknüpfte Nukleotidsequenz, wobei die Nukleotidsequenz für das Polypeptid codiert,
(d) eine operativ mit der Nukleotidsequenz verknüpfte Polyadenylierungsstelle (PAA),
wobei es sich bei dem Promoter nicht um einen natürlichen FAD2-Promoter handelt und wobei das Intron zwischen dem Promoter und dem Translationsursprung der Nukleotidsequenz insertiert ist.

2. Genkonstrukt nach Anspruch 1, wobei es sich bei dem Promoter um einen gewebespezifischen Promoter handelt.

3. Genkonstrukt nach Anspruch 1, wobei es sich bei dem Promoter um einen konstitutiven Promoter handelt.

4. Genkonstrukt nach einem der Ansprüche 1 bis 3, das zur Integration in das Genom der Pflanzenzelle fähig ist.

5. Verfahren zur Verbesserung der Expression eines Polypeptids in einer Pflanzenzelle, umfassend den Schritt, daß man das Genkonstrukt nach einem der Anspüche 1 bis 4 in die Pflanzenzelle einführt.

6. Verfahren zur Erhöhung der Expressionsrate eines Gens unter der Kontrolle eines Promoters in einer Pflanzenzelle, wobei das Verfahren die folgenden Schritte umfaßt:
(a) Insertieren des ersten Introns des FAD2-Gens, wobei das FAD2-Gen aus Arabidopsis thaliana stammt und wobei das erste Intron SEQ ID NO. 1 entspricht, zwischen den Promoter und den Translationsursprung des Gens, wodurch ein intronmodifiziertes Gen gebildet wird; und
(b) Einführen des intronmodifizierten Gens in eine Pflanzenzelle, so daß das Gen unter der Kontrolle des Introns in der Pflanzenzelle exprimiert wird, wobei die Expressionsrate des Gens im Vergleich zu einem unmodifizierten Gen erhöht ist,
wobei es sich bei dem Promoter nicht um einen natürlichen FAD2-Promoter handelt.

7. Verwendung des ersten Introns des FAD2-Gens in Kombination mit einem Promoter, der sich von dem natürlichen FAD2-Promoter unterscheidet, zur Verbesserung der Expression eines Polypeptids in einer Pflanzenzelle, wobei das FAD2-Gen aus Arabidopsis thaliana stammt und wobei das erste Intron SEQ ID NO. 1 entspricht.

8. Pflanze, umfassend ein Genkonstrukt nach Anspruch 1, das in ihr Genom integriert ist.

9. Pflanze nach Anspruch 8, wobei es sich um eine monokotyle Pflanze handelt.

10. Pflanze nach Anspruch 9, ausgewählt aus der Gruppe bestehend aus Weizen, Gerste, Mais und Reis.

## Revendications

1. Construction génétique pour l'expression d'un polypeptide dans une cellule végétale, comprenant :
(a) un promoteur opérationnel dans ladite cellule végétale,
(b) le premier intron du gène FAD2, ledit gène FAD2 provenant d'Arabidopsis thaliana et ledit premier intron étant représenté par SEQ ID NO : 1,
(c) une séquence nucléotidique liée de manière opérationnelle audit promoteur, ladite séquence nucléotidique codant pour ledit polypeptide,
(d) un site de polyadénylation (PAA) lié de manière opérationnelle à ladite séquence nucléotidique
ledit promoteur n'étant pas un promoteur naturel de FAD2 et ledit intron étant inséré entre ledit promoteur et le site d'initiation de la traduction de ladite séquence nucléotidique.

2. Construction génétique selon la revendication 1, dans laquelle ledit promoteur est un promoteur tissu-spécifique.

3. Construction génétique selon la revendication 1, dans laquelle ledit promoteur est un promoteur constitutif.

4. Construction génétique selon l'une quelconque des revendications 1 à 3, susceptible de s'intégrer au sein du génome de ladite cellule végétale.

5. Méthode permettant d'amplifier l'expression d'un polypeptide dans une cellule végétale, comprenant l'étape consistant à introduire la construction génétique selon l'une quelconque des revendications 1 à 4 dans ladite cellule végétale.

6. Méthode permettant d'accroître, dans une cellule végétale, le taux d'expression d'un gène sous le contrôle d'un promoteur, ladite méthode comprenant les étapes consistant à :
(a) insérer le premier intron du gène FAD2, ledit gène FAD2 provenant d'Arabidopsis thaliana et ledit premier intron étant représenté par SEQ ID NO : 1, entre ledit promoteur et le site d'initiation de la traduction dudit gène, formant de ce fait un gène modifié par un intron ; et
(b) introduire ledit gène modifié par un intron dans une cellule végétale de telle sorte que ledit gène est exprimé sous le contrôle dudit intron dans ladite cellule végétale, ce par quoi le taux d'expression dudit gène est accru par rapport à un gène non modifié,
ledit promoteur n'étant pas un promoteur naturel de FAD2.

7. Utilisation du premier intron du gène FAD2 en association avec un promoteur différent du promoteur naturel de FAD2, pour amplifier l'expression d'un polypeptide dans une cellule végétale, ledit gène FAD2 provenant d'Arabidopsis thaliana et ledit premier intron étant représenté par SEQ ID NO : 1.

8. Plante comprenant une construction génétique selon la revendication 1 intégrée dans son génome.

9. Plante selon la revendication 8, qui est une monocotylédone.

10. Plante selon la revendication 9, qui est choisie dans le groupe constitué du blé, de l'orge, du maïs et du riz.
